(19) Europäisches Patentamt / European Patent Office / Office européen des brevets

(11) **EP 4 180 032 A1**

(12) **EUROPEAN PATENT APPLICATION**

(43) Date of publication:
**17.05.2023  Bulletin 2023/20**

(21) Application number: **21208639.1**

(22) Date of filing: **16.11.2021**

(51) International Patent Classification (IPC):
**A61K 31/04** (2006.01)  **A61K 31/16** (2006.01)
**A61K 31/198** (2006.01)  **A61K 31/728** (2006.01)
**A61K 47/61** (2017.01)  **A61K 47/64** (2017.01)
**A61P 21/00** (2006.01)  **A61P 25/00** (2006.01)
**A61P 29/00** (2006.01)

(52) Cooperative Patent Classification (CPC):
**A61K 31/728; A61K 31/04; A61K 31/16;
A61K 31/198; A61K 47/61; A61K 47/6455;
A61P 21/00; A61P 25/00; A61P 29/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **National University of Ireland Galway
Galway (IE)**

(72) Inventors:
• **LIDDY, Alison**
  **Galway (IE)**
• **MITRA, Tapas**
  **Galway (IE)**
• **DUFFY, Garry**
  **Galway (IE)**
• **QUINLAN, Leo**
  **Galway (IE)**
• **MCDERMOTT, Barry**
  **Galway (IE)**
• **FINN, David**
  **Galway (IE)**
• **FERDOUSI, Mehnaz**
  **Galway (IE)**

(74) Representative: **Barker Brettell LLP
100 Hagley Road
Edgbaston
Birmingham B16 8QQ (GB)**

Remarks:
Claims 16, 17, 18, 22 & 23 are deemed to be abandoned due to non-payment of the claims fees (Rule 45(3) EPC).

(54)  **NERVE AND EXCITABLE TISSUES MODULATION TREATMENT**

(57)  The invention relates to a peptide drug for use in the localised modulation of nerve cell activity, cardiomyocyte or muscle cell electrical activity in a subject, wherein the peptide drug comprises a polymer in a carrier, and further comprising: i) a plurality of cationic peptides, or an amino acid monomer thereof, covalently bound to the polymer; and/or ii) a plurality of cationic peptides, or an amino acid monomer thereof, mixed with the polymer. The invention further related to the use of CARPs for use in the localised modulation of nerve cell activity, cardiomyocyte or muscle cell electrical activity in a subject. The invention further relates to modulation of chondrocyte function.

**EP 4 180 032 A1**

Figure 1

**Description**

This invention relates to therapies related to modulation of excitable tissue activity. These tissues include nerve and muscle, and for use in conditions including pain, inappropriate motor neuron activity, and cardiac arrhythmia for example, in a subject and associated compositions, uses and methods of manufacture.

The rationale for use of this invention in pain and in particular osteoarthritis is now briefly described.

Pain can be classified as nociceptive, inflammatory, neuropathic, or functional. In all cases the sensation of pain is caused by the relaying of electrical signals along afferent pain sensing neurons to the brain where the signal is interpreted.

Nociceptive pain is a type of pain caused by damage to body tissue, which can be caused by an external injury or a condition such as osteoarthritis where joint structure and tissue is degraded.

Osteoarthritis (OA) is an incurable complex disease impacting 350 million people worldwide, including 40 million in Europe. OA is a degenerative joint disease where progressive degradation of bone and cartilage in the affected joint results in severe pain, reduced joint function, and stiffness.

Osteoarthritis most commonly affects the knee joint, representing 45% of cases (18 million Europeans). Knee OA is the 4th leading cause of disability worldwide, with rapid increasing prevalence due to an ageing population and the growing obesity epidemic. Knee osteoarthritis affects more people globally each year than cancer and heart disease. There is no means to stop disease progression, and no long-lasting effective pain relief treatment. Osteoarthritis pain is particularly severe impacting patients' sleep, mood, and everyday life. Patients with osteoarthritis suffer from chronic pain for an average of 28 years from initial diagnosis, as well as suffering immobility. Inadequate management of chronic pain is directly related to quality of life with one third of people with osteoarthritis over 45 suffering with high levels of depression and sleep deprivation.

Knee osteoarthritis is a painful, debilitating, and progressive disease where no current treatment offers patients effective pain relief without side effects. People living with knee osteoarthritis describe their pain as continuous, sharp, stabbing and burning. Current treatment options for knee osteoarthritis patients fall into four categories, with the patient pathway typically progressing through these options with disease progression:

Oral Medications: These include non-steroidal anti-inflammatory drugs (NSAIDs) and opioids. Both these classes of drugs manage the symptoms but are short acting usually requiring daily dosing and have serious side-effect. NSAIDs cannot be used long-term due to toxicity to the stomach, liver, and kidneys. Opioids have a pernicious effect, tolerance resulting in ever increasing doses needed to control pain, and dependency leading to addiction. The opioid crisis in the US is testament to these problems, and indeed osteoarthritis patients constitute the largest group of opioid users. Furthermore, as no agent stops progression of osteoarthritis, stronger doses are needed as the disease progresses.

Steroid Injectables: Steroids are injected directly into the intra-articular space of the joint to give pain relief. However, the effect lasts for an average of only 2-4 weeks, and irreplaceable cartilage is destroyed each time steroids are used. Hence, these agents are restricted in use to a maximum of four treatments per year, with patients invariably reliant on oral medications between injections.

Hyaluronic Acid (HA) Injectables: HA is a major component of the fluid in a normal healthy joint, acting as a lubricant and shock absorber. Commercial HA injectables replace HA in the damaged joint and while safe, only provide mild pain relief through a barrier effect. This lack of efficacy has resulted in The American Academy of Orthopaedic Surgeons (AAOS) and NICE (UK) no longer recommending HA as a treatment for osteoarthritis.

Stem Cell and Platelet Injectables: Although currently an area of active research, these products have failed do demonstrate that they are effective in relieving OA pain.

Knee Replacement Surgery: Approximately 5.6% of patients every year opt for knee replacement surgery. This is an end-stage treatment where the damaged knee is replaced with an artificial metal implant. This is an expensive highly invasive procedure, that is often associated with a high failure rate and need for revision. Hence, surgery is considered a last-resort treatment for patients with terminally damaged knee joints and not responding to other treatment options.

In addition to application in OA conditions, it is recognised that agents that can be used to modulate the activity of pain sensing neurons may also be useful for other painful conditions such as those featuring neuropathic pain, Painful Bladder Syndrome, and Vaginal Atrophy.

These agents may also modulate the activity of motor neurons which would be useful as a treatment in conditions such as spasticity, trigger point pain, migraine, dystonia, essential tremor, which all feature motor nerve overactivity, and in conditions such as myasthenia gravis which feature motor neuron underactivity. In addition, these agents would have utility in cosmetic applications benefiting from motor nerve modulation.

In all cases it would be desirable that a therapy offer immediate, long-lasting, and safe modulation of nerve cell activity and in the nerve cell type of interest (pain or motor for example).

Like neurons, muscle cells are classified as excitable and respond to electrical stimulation, which can be modulated to control function. Muscle cells can be classified as skeletal, smooth, and cardiac. Muscle cells are innervated and so modulation of function can be achieved by effect on the nerves serving them (as is the case with motor neurons to skeletal muscles). However, the muscle cell itself can alternatively be the target of modulation.

Taking cardiac muscle as an example, these cell types (cardiomyocytes) can be divided into specialist worker cells and pacemaker cells. Worker cardiomyocytes are responsible for the mechanical events of the cardiac cycle (relaxation allowing filling, and contraction resulting in ejection of blood into the arteries). The pacemaker cells are responsible for the electrical events of the cardiac cycle and ensure the worker cells are stimulated in a coordinated manner.

Modulation of cardiomyocyte activity would be advantageous in conditions such as: Heart failure - treatments that result in a slower (negative chronotropic) but stronger (positive inotropic) heart contraction is of benefit in heart failure; Arrhythmias - treatments that suppress or block aberrant pacemaker cells are of value in these conditions. Arrythmias include conditions such as atrial fibrillation, ventricular fibrillation, Wolff-Parkinson-White syndrome; Cardiac myopathies - where modulation of mechanical or contractile cardiac cycles are of value in treatment (for example dilated cardiomyopathy).

According to a first aspect of the present invention, there is provided a peptide drug for use in the localised modulation of nerve cell activity, cardiomyocyte or muscle cell electrical activity in a subject, wherein the peptide drug comprises a polymer in a carrier, and further comprising:

i) a plurality of cationic peptides, or an amino acid monomer thereof, covalently bound to the polymer; and/or

ii) a plurality of cationic peptides, or an amino acid monomer thereof, mixed with the polymer.

According to another aspect of the present invention, there is provided a peptide drug comprising a polymer in a carrier, and further comprising:

i) a plurality of cationic peptides, or an amino acid monomer thereof, covalently bound to the polymer; and/or

ii) a plurality of cationic peptides, or an amino acid monomer thereof, mixed with the polymer.

Cationic peptides, such as Cationic Arginine-Rich Peptides (CARPs), are reported to have multi-modal properties. However, the clinical use of CARPs has proven limited to date due to the fact these molecules are rapidly broken down *in-vivo* and longer chain CARPs have proven to be toxic. Advantageously, the present invention provides a peptide drug in the form of cationic peptides, or an amino acid monomer thereof, that are mixed with, and/or covalently bound to, a polymer base (which may be termed herein as "Hydrobloc"), it has been proven herein to reduce ion flow into excitable cells and to reduce the ability of these cells to generate action potentials. This reduction in action potential generation translates into pain relief when applied to pain sensing neurons (which has been demonstrated *in-vivo),* reduced skeletal muscle activity when applied to motor neurons or skeletal muscle cells, and modulation of cardiac activity when applied to cardiomyocytes.

**The Polymer**

The polymer may be natural or synthetic. The polymer may be biocompatible. In a preferred embodiment, the polymer is a biodegradable polymer. The polymer may be non-toxic to mammalian subjects at therapeutically acceptable levels.

In one embodiment, the polymer is selected from one or more of poly-ethylene glycol (PEG), poly(lactic-co-glycolic acid) (PLGA), polylactic acid, polysaccharides, silica, hyaluronic acid or an ester or salt thereof, polycaprolactone (PCL), polyamides and poly(ester-amide)s, polyurethanes, polyanhydrides, polyvinyl alcohol, chitin, cellulose, alginic acid or alginate, poly(hydroxybutyrate) (PHB), starch and its modified polymers, dendrimers, collagen, gelatin, calcium hydroxyapatite, polyethylene glycol diacrylate, sodium hyaluronate, polyglycolide, poly-3-hydroxybutyrate, and chitosan; or combinations thereof.

The polymer may comprise or consist of glycosaminoglycans. In one embodiment, the polymer comprises, or consists of, hyaluronic acid (HA) or sodium hyaluronate. Hyaluronic acid (HA) may also be known as hyaluronan.

In another embodiment the polymer comprises polyethylene glycol (PEG).

The polymer may comprise or consist of a polymer selected from any of the group comprising polyacrylamide; pectin; alginate; carboxymethylcellulose; methylcellulose; PLGA; PEG; polysaccharide, such as starch, cellulose, chitin, alginate, hyaluronate; proteins such as collagen, gelatin, casein, albumin; polyvinyl alcohol (PVA); polyvinylpyrrolidone (PVP); polyethyleneglycol (PEG); polylactic acid; and polyhydroxy acid (PHA), or combinations thereof. The polymer may comprise or consist of a polymer selected from any of the group comprising poly ([alpha]-hydroxyacids) including poly-lactide-co-glycolide (PLGA), poly-lactic acid, polyethyleneimine (PEI), polylactic or polyglycolic acids, poly-lactide poly-glycolide copolymers, and poly-lactide poly-glycolide polyethylene glycol copolymers, polyethylene glycol (PEG), polyesters, poly ([epsilon]-caprolactone), poly (3- hydroxybutyrate), poly (s-caproic acid), poly (p-dioxanone), poly (propylene fumarate), poly (orthoesters), polyol/diketene acetals addition polymers, polyanhydrides, poly (sebacic anhydride) (PSA), poly (carboxybiscarboxyphenoxyphosphazene) (PCPP), poly [bis(p-carboxyphenoxy) methane] (PCPM), poly (amino acids), poly (pseudo amino acids), polyphosphazenes, derivatives of poly [(dichloro) phosphazene], poly [(organo) phosphazenes], polyphosphates, polyethylene glycol polypropylene block co-polymers for example that sold under the trade mark Pluronics(TM), natural or synthetic polymers such as silk, elastin, chitin, chitosan, fibrin, fibrinogen, polysaccharides (including pectins), alginates, collagen, peptides, polypeptides or proteins, copolymers prepared from the monomers of any of these polymers, random blends of these polymers, any suitable polymer and mixtures or combinations thereof. In another embodiment, the polymer may comprise or consist of a carbohydrate polymer such as dextran.

The polymer may have a molecular weight sufficient to form a gel under normal environmental conditions, for example at ambient room temperature (i.e. about 24°C). In another embodiment, the polymer may have a molecular weight sufficient to form a solution or a colloid with a carrier (for example an aqueous carrier) under normal environmental conditions, for example at room temperature (i.e. about 24°C). The polymer may be at a sufficient concentration and have a sufficient molecular weight to form a solution or a colloid or a gel with the carrier, under normal environmental conditions for example at room temperature (i.e. about 24°C).

The polymer may have a molecular weight of at least 5 kDa. Alternatively, the polymer may have a molecular weight of at least 10 kDa. Further alternatively, the polymer may have a molecular weight of at least 20 kDa. Alternatively, the polymer may have a molecular weight of at least 50 kDa. The polymer may have a molecular weight of up to 3000 kDa. Alternatively, the polymer may have a molecular weight of up to 2200 kDa. In one embodiment, the polymer has a molecular weight of between about 5 kDa and about 3000 kDa. In another embodiment, the polymer has a molecular weight of between about 10 kDa and about 3000 kDa. In another embodiment, the polymer has a molecular weight of between about 20 kDa and about 3000 kDa. In another embodiment, the polymer has a molecular weight of between about 50 kDa and about 2200 kDa. In one embodiment, the polymer has a molecular weight of between about 50 kDa and about 3000 kDa. In an alternative embodiment, the polymer has a molecular weight of between about 50 kDa and about 2500 kDa. In an alternative embodiment, the polymer has a molecular weight of between about 50 kDa and about 2400 kDa. In an alternative embodiment, the polymer has a molecular weight of between about 500 kDa and about 2400 kDa. In one embodiment, the polymer has a molecular weight of between 2.2 MDa and 2.4 MDa.

In an embodiment wherein the polymer is hyaluronic acid, the polymer may have a molecular weight of about 2200 kDa. In an embodiment wherein the polymer is PEG, the polymer may have a molecular weight of about 20 kDa or 50 kDa.

The polymer may be cross-linked, for example to form a gel, or in another embodiment, the polymer may not require cross-linking. The polymer cross-linking may be covalent cross-linking or ionic or a blend of both, for example via charged functional moieties bound to the polymer.

The polymer, such as hyaluronic acid, may be provided in a concentration of up to 30% w/v in the carrier. The polymer, such as hyaluronic acid or PEG, may be provided in a concentration of between 10 mg/ml and 20mg/ml. In another embodiment, the polymer, such as hyaluronic acid or PEG, may be provided in a concentration of between 0.4 mg/ml and 30 mg/ml. In another embodiment, the polymer, such as hyaluronic acid or PEG, may be provided in a concentration of between 10 mg/ml and 30mg/ml.

**Covalent Attachment / Linking**

The skilled person will be familiar with methods of covalently linking the cationic peptides, or an amino acid monomer thereof, to the polymer. In one embodiment, the cationic peptides, or an amino acid monomer thereof, are covalently linked to the polymer by an amide bond. In another embodiment, the cationic peptides, or an amino acid monomer thereof, are covalently linked to the polymer by esterification. In another embodiment, the cationic peptides, or an amino acid monomer thereof, are covalently linked to the polymer by carbodiimide coupling.

The cationic peptides, or an amino acid monomer thereof, may be covalently linked to the polymer, such as HA or PEG, through covalent linkage using carbodiimide chemistry.

The cationic peptides, or an amino acid monomer thereof, may be anchored/linked to the polymer by chemical bonding via a linker. The linker may comprise a chemical bond between atoms, such as C-C, C=C, C(O)-NH, C-O, C-Si, or C-S. The cationic peptides, or an amino acid monomer thereof, may be anchored to the polymer by a disulphide bond.

Advantageously, these bonds can be characterised by different stability under different environmental conditions. Thus, by inclusion of chemically labile bonds (such as a disulphide bond) one can engineer bioresponsive gel materials that can be disassembled upon certain conditions (as for example 1000-fold higher concentration of thiols in cellular cytoplasm as compared with extracellular environment).

The linker molecule may be an organic molecule. The linker molecule may be selected from any of the group comprising acrylamide, alkane, alkene, alkyne, alcohol, aldehyde, ketone, amino acid, ester, cycloalkane, sugar, and nucleic acid.

The linker molecule may be arranged to be anchored to the polymer by chemical reaction. The chemical reaction may be spontaneous (i.e. self-catalysed), chemically catalysed or photo-catalysed. The linker molecule may comprise a thiol moiety for linking the linker molecule to the polymer by chemical reaction. The linker molecule may comprise a disulphide linked pyridine for linking the linker molecule to the polymer by chemical reaction. The polymer may comprise a disulphide linked pyridine, or a thiol moiety for linking the polymer to the linker molecule by chemical reaction.

A benefit of covalently linking the cationic peptide, or an amino acid monomer thereof, to the polymer is that it can remain *in situ,* such as in a joint, for longer, giving long lasting effects such as pain relief. The peptide without the polymer is quickly broken down and removed from the joint. Advantageously, the linking of the peptides, or an amino acid monomer thereof, to the polymer can provide a therapy that has a clinically acceptable injection frequency, such as 2-4 times per year. The polymer linking may reduce degradation and prevent escape of the peptide by trans-synovial flux in a joint. A HA polymer backbone may be protected from hyaluronidase degradation and mechanical degradation from compression. The specific distribution of the peptide, or an amino acid monomer thereof, can avoid non-targeted biodistribution. Furthermore, the peptide drug may provide the benefit of charge maintenance, where it stops electrostatic charge loss in the joint.

Furthermore, longer chain CARPs ($\geq$ 40 kDa in size) have proven to have limited uses as therapeutics due the toxic effects. Advantageously, the linking of the longer chain peptides, to the polymer can provide a therapy that is safe where the toxic properties of the peptide have been removed.

**The Cationic Peptide and Amino Acid Monomer Thereof**

A cationic peptide is understood to mean a single amino acid residue or polymer of amino acid residues where the overall peptide has a positive or polar positive charge (i.e., is cationic).

The cationic peptide may comprise or consist of at least two amino acid residues, such as two arginine residues, or a larger chain of repeating arginine units such as poly-arginine. The monomer of a cationic peptide may consist of a single amino acid residue that is positively charged, for example arginine. The cationic peptide may be a CARP (Cationic Arginine Rich Peptide). In one embodiment, the cationic peptide comprises a majority (i.e., greater than 50%) positively charged amino acid residues, such as a majority of arginine residues. In another embodiment, the cationic peptide comprises at least 60% positively charged amino acid residues, such as at least 60% arginine residues. In another embodiment, the cationic peptide comprises at least 80% positively charged amino acid residues, such as at least 80% arginine residues. In another embodiment, the cationic peptide comprises at least 90% positively charged amino acid residues, such as at least 90% arginine residues. In another embodiment, the cationic peptide comprises at least 95% positively charged amino acid residues, such as at least 95% arginine residues.

Alternatively, the cationic peptide may comprise or consist of lysine, such as poly-lysine. In one embodiment, the cationic peptide comprises a majority (i.e. greater than 50%) of lysine residues. In another embodiment, the cationic peptide comprises at least 60% lysine residues. In another embodiment, the cationic peptide comprises at least 80% lysine residues. In another embodiment, the cationic peptide comprises at least 90% lysine residues. In another embodiment, the cationic peptide comprises at least 95% lysine residues.

Alternatively, the cationic peptide may comprise or consist of histidine, such as poly-histidine. In one embodiment, the cationic peptide comprises a majority (i.e. greater than 50%) of histidine residues. In another embodiment, the cationic peptide comprises at least 60% histidine residues. In another embodiment, the cationic peptide comprises at least 80% histidine residues. In another embodiment, the cationic peptide comprises at least 90% histidine residues. In another embodiment, the cationic peptide comprises at least 95% histidine residues.

Alternatively, the cationic peptide may comprise or consist of arginine and lysine residues. In one embodiment, the cationic peptide comprises a majority (i.e., greater than 50%) of arginine and lysine residues. In another embodiment, the cationic peptide comprises at least 60% arginine and lysine residues. In another embodiment, the cationic peptide comprises at least 80% arginine and lysine residues. In another embodiment, the cationic peptide comprises at least 90% arginine and lysine residues. In another embodiment, the cationic peptide comprises at least 95% arginine and lysine residues.

Alternatively, the cationic peptide may comprise or consist of histidine and/or arginine and/or lysine residues. In one embodiment, the cationic peptide comprises a majority (i.e., greater than 50%) of histidine and arginine and/or lysine residues. In another embodiment, the cationic peptide comprises at least 60% histidine and arginine and/or lysine residues. In another embodiment, the cationic peptide comprises at least 80% histidine and arginine and/or lysine residues. In another embodiment, the cationic peptide comprises at least 90% histidine and arginine and/or lysine residues. In another embodiment, the cationic peptide comprises at least 95% histidine and arginine and/or lysine residues.

Alternatively, the cationic peptide may comprise or consist of other amino acid residues such as ornithine, asparagine or glutamine that are cationic or polar-cationic in nature. Further such residues can be naturally occurring or synthetic in nature. In one embodiment, the cationic peptide comprises a majority (i.e. greater than 50%) of such cationic residues. In another embodiment, the cationic peptide comprises at least 60% of such cationic residues. In another embodiment, the cationic peptide comprises at least 80% of such cationic residues. In another embodiment, the cationic peptide comprises at least 90% of such cationic residues. In another embodiment, the cationic peptide comprises at least 95% of such cationic residues.

The cationic peptide, such as poly-arginine, may have a molecular weight in the range of 1,900-70,000 Da, optionally in the range of 15,000-70,000 Da.

In one embodiment, the cationic peptide, such as poly-arginine, has a molecular weight of about 1.9kDa. In one embodiment, the cationic peptide, such as poly-arginine, has a molecular weight of between about 1 kDa and about 100 kDa. In another embodiment, the cationic peptide, such as poly-arginine, may have a molecular weight in the range of 1 kDa to 70 kDa. In another embodiment, the cationic peptide, or monomer thereof, may have a molecular weight in the range of 174 Da to 70 kDa. In another embodiment, the cationic peptide, such as poly-arginine, has a molecular weight of between about 1.5kDa and about 70kDa. In another embodiment, the cationic peptide, such as poly-arginine, has a molecular weight of between about 1.9kDa and about 70kDa. In another embodiment, the cationic peptide, such as poly-arginine, may have a molecular weight in the range of 1.9 to 30 kDa. In another embodiment, the cationic peptide, or monomer thereof, may have a molecular weight in the range of 1 kDa to 30 kDa. In another embodiment, the cationic peptide, such as poly-arginine, may have a molecular weight in the range of 1.9 to 15 kDa. In another embodiment, the cationic peptide, such as poly-arginine, has a molecular weight of between about 1.5kDa and about 10kDa. In another embodiment, the cationic peptide, such as poly-arginine, has a molecular weight of between about 1.9kDa and about 9.6kDa. In another embodiment, the cationic peptide, or monomer thereof, may have a molecular weight in the range of 174Da to 30 kDa. In another embodiment, the cationic peptide, or monomer thereof, may have a molecular weight in the range of 174Da to 15 kDa.

In one embodiment the peptide drug comprises or consists of PEG polymer covalently linked to poly-arginine or poly-lysine; or combinations thereof. In another embodiment the peptide drug comprises or consists of hyaluronic acid polymer covalently linked to poly-arginine or poly-lysine; or combinations thereof. In another embodiment the peptide drug comprises or consists of hyaluronic acid polymer covalently linked to arginine monomer or lysine monomer; or combinations thereof.

In one embodiment, the cationic peptide, such as poly-arginine, has a molecular weight of about 1.9kDa, and the polymer has a molecular weight of between 2.2MDa and 2.4MDa. In one embodiment, the cationic peptide, such as poly-arginine, has a molecular weight of between about 1kDa and about 100kDa, and the polymer has a molecular weight of between 2.2MDa and 2.4MDa. In another embodiment, the cationic peptide, such as poly-arginine, has a molecular weight of between about 1.5kDa and about 70kDa, and the polymer has a molecular weight of between 2.2MDa and 2.4MDa. In another embodiment, the cationic peptide, such as poly-arginine, has a molecular weight of between about 1.9kDa and about 70kDa, and the polymer has a molecular weight of between 2.2MDa and 2.4MDa. In another embodiment, the cationic peptide, such as poly-arginine, has a molecular weight of between about 1.5kDa and about 10kDa, and the polymer has a molecular weight of between 2.2MDa and 2.4MDa. In another embodiment, the cationic peptide, such as poly-arginine, has a molecular weight of between about 1.9 kDa and about 9.6 kDa, and the polymer has a molecular weight of between 2.2MDa and 2.4MDa.

The combined molecular weight of the polymer and covalently bound cationic peptides may be between 500 kDa and 3000 kDa.

In one embodiment, the polymer is covalently linked to the peptide in a gram:gram ratio of about 1: 0.001 polymer to peptide. In another embodiment, the polymer comprises consists of HA and/or PEG covalently linked to the peptide (e.g. peptide size 1.5 kD - 70 kD or 1.5 kDa - 30 kDa) in a gram:gram ratio of about 1: 0.001 polymer to peptide. In another embodiment, the polymer comprises consists of HA and/or PEG covalently linked to the peptide, wherein the peptide comprises consists of poly-arginine (e.g. peptide size 1.5 kD - 70 kD) in a gram:gram ratio of about 1: 0.5 polymer to peptide. In another embodiment, the polymer comprises consists of HA and/or PEG covalently linked to the peptide, wherein the peptide comprises consists of poly-arginine (e.g. peptide size 1.5 kD - 30 kD) in a gram:gram ratio of about 1: 5 polymer to peptide. In another embodiment, the polymer comprises consists of HA and/or PEG covalently linked to the peptide, wherein the peptide comprises consists of poly-arginine (e.g. peptide size 1.5 kD - 30 kD) in a gram:gram ratio of between about 1: 0.5 and about 1: 5 polymer to peptide.

In one embodiment, the polymer is mixed with peptide in a gram:gram ratio of about 1: 0.001 polymer to peptide. In another embodiment, the polymer comprises consists of HA and/or PEG mixed with the peptide (e.g. peptide size 1.5 kD - 70 kD or 1.5 kDa - 30 kDa) in a gram:gram ratio of about 1: 0.001 polymer to peptide. In another embodiment, the polymer comprises consists of HA and/or PEG mixed with peptide, wherein the peptide comprises consists of poly-arginine (e.g. peptide size 1.5 kD - 70 kD) in a gram:gram ratio of about 1 : 0.5 polymer to peptide. In another embodiment, the polymer comprises consists of HA and/or PEG mixed with peptide, wherein the peptide comprises consists of poly-arginine (e.g. peptide size 1.5 kD - 30 kD) in a gram: gram ratio of about 1: 5 polymer to peptide. In another embodiment, the polymer comprises consists of HA and/or PEG mixed with peptide, wherein the peptide comprises consists of poly-arginine (e.g. peptide size 1.5 kD - 30 kD) in a gram : gram ratio of between about 1 : 0.001 and about 1 : 5, or more, polymer to peptide. In another embodiment, the polymer comprises consists of HA and/or PEG mixed with peptide, wherein the peptide comprises consists of poly-arginine (e.g. peptide size 1.5 kD - 30 kD) in a gram: gram ratio of between about 1: 0.001 and about 1:10 polymer to peptide.

In another embodiment, the polymer is mixed (non-covalently bound) and covalently linked with the peptide in a gram: gram ratio of about 1 : 0.001 polymer to peptide. In another embodiment, the polymer is mixed (non-covalently bound) and covalently linked with the peptide in a gram: gram ratio of about 1:0.002 polymer to peptide. In another embodiment, the polymer is mixed (non-covalently bound) and covalently linked with the peptide in a gram:gram ratio of about 1:0.05 polymer to peptide. In another embodiment, the polymer is mixed (non-covalently bound) and covalently linked with the peptide in a gram: gram ratio of about 1:1 polymer to peptide. In another embodiment, the polymer is mixed (non-covalently bound) and covalently linked with the peptide in a gram : gram ratio of about 1 : 2.5 polymer to peptide. In another embodiment, the polymer is mixed (non-covalently bound) and covalently linked with the peptide in a gram: gram ratio of between about 1:1 and about 1 : 0.05 polymer to peptide. In another embodiment, the polymer is mixed (non-covalently bound) and covalently linked with the peptide in a gram: gram ratio of between about 1:0.001 and about 1 : 2.5 polymer to peptide.

In one embodiment, the peptide drug may comprise a combination of peptides covalently linked to the polymer and/ or a mixture of peptides (i.e. not covalently bound to the polymer). In one embodiment, the polymer is both covalently linked to the peptide and/ or mixed with the peptide (e.g. peptide size 1.5 kD - 70 kD or 1.5 kDa-30 kDa) in a gram: gram ratio in the range of 1 : 0.001 : 0.001 to 1: 5 : 5 polymer : covalently linked peptide : free (non-covalently bound) peptide. In one embodiment, the polymer comprises or consists of HA and/or PEG, and is both covalently linked to the peptide, and/ or mixed with the peptide (e.g. peptide size 1.5kD-70 kD or 1.5kDa-30 kDa) in a gram:gram ratio of about 1 : 0.001 : 0.001 to 1: 5 : 5 polymer: covalently linked peptide : free (non-covalently bound) peptide. In one embodiment, the polymer comprises or consists of HA and/or PEG, and is both covalently linked to the peptide, which comprises or consists of poly-lysine, and/ or mixed with the peptide (e.g. peptide size 1.5 kD - 70 kD or 1.5 kDa-30 kDa) which comprises or consist of PL, in a gram:gram ratio of about 1 : 0.001:0.001 to 1: 5 : 5 polymer: covalently linked peptide : free (non-covalently bound) peptide.

## Composition Properties

The peptide drug may be in the form of a gel, a colloid or a solution. In a preferred embodiment, the peptide drug is a gel, for example as formed by the polymer.

The gel may have a zeta-potential in the range of -38 mV to + 35 mV; alternatively in the range of -20 to + 20 mV, further alternatively about -1 mV. In one embodiment, the gel has a zeta-potential in the range of about -23 to -1 mV. In another embodiment, the gel has a zeta-potential in the range of about -12 to -1 mV. In another embodiment, the gel has a zeta-potential in the range of about -23 to -16 mV. In another embodiment, the gel has a zeta-potential in the range of about -23 to +5 mV.

The gel may have a concentration dependent dynamic viscosity of between $10^1$ and $10^6$ mPa.S.

Advantageously, the provision of the peptide drug in the form of the gel may provide the additional benefit of providing similar properties, such as viscosity and rheology to natural joint synovial fluid and HA, which can contribute to joint function, joint pain relief and sustained pain relief, where the molecule takes longer to be removed and broken down from the joint.

## The Carrier and Other Additives

The carrier may be a pharmaceutically acceptable carrier. The carrier may be a liquid carrier. The carrier may be fluid based for example as an aqueous, organic, or multiphasic liquid, or may be solid in nature. The carrier may be a gel or a paste. In one embodiment, the carrier is an aqueous carrier.

In one embodiment, the carrier is aqueous and comprises of water or saline or a buffer or buffered saline.

The carrier may further comprise a further active agent. The active agent may be a therapeutically, prophylactically or diagnostically active substance. The active agent may be a bioactive substance. The active agent may be selected from the group comprising a drug, pro-drug, peptide, protein, and nucleic acid, or combinations thereof. The active agent may comprise or consist of a biomolecule.

The peptide drug may be formulated for administration by a pharmaceutically or cosmetically acceptable route such as by injection or by topical application. The peptide drug may further comprise pharmaceutically acceptable excipients. The peptide drug may further comprise an excipient selected from the group consisting of pharmaceutically acceptable salts, polysaccharides, peptides, proteins, amino acids, synthetic polymers, natural polymers, and surfactants.

**The Cells**

In one embodiment, peptide drug is for localised modulation of nerve cell activity. Nerve cells may comprise cortical neurons, efferent neurons such as motor neurons, or afferent neurons such as pain-sensing nerves cells and DRGs.

In another embodiment, the peptide drug is for localised modulation of cardiomyocyte or muscle cell electrical activity. Cardiomyocytes may comprise specialist contractile cells and/or specialist pacemaker cells. Muscle cells may comprise one or more of cardiomyocytes, skeletal muscle cell, smooth muscle cell.

**The subject**

The subject may be a mammal. The subject may be human. The subject may have abnormal physiology/anatomy including tissue damage or tissue degeneration. The subject may have a bone fracture or degeneration. In one embodiment, the subject has osteoarthritis, for example of a joint, such as the knee, hip, shoulder, elbow or fingers.

The subject may be suffering from increased neural activity selected from pain, hypertension, hyperhidrosis, and involuntary muscle spasms including dystonia. The subject may be suffering from spasticity, migraine, dystonia or essential tremor. The subject may be suffering from a pain, which is not neuropathic pain. The subject may be suffering from nociceptive pain. The subject may be suffering from a condition that requires modulation of excitable tissues such as neural and/ or muscle cells including cardiac arrhythmias or skeletal muscle overactivity.

In another embodiment, the subject may be suffering from pain from neuropathic pain, painful bladder syndrome, cystitis, or vaginal atrophy.

In another embodiment, the subject may have normal physiology/ anatomy which it is desirable to modify, for example if undergoing or in need of cosmetic treatment.

**Administration**

In one embodiment, the peptide drug is, or is arranged to be, administered to the subject by local injection, for example at the site of a pain, such as in a joint or tissue. The administration may be targeted to a specifically targeted nerve or muscle tissue. The target nerve may be an afferent nerve associated with a target site; the target site and associated afferent nerve being selected from:

- Knee Joint -Genicular nerve or branches thereof;

- Hip Joint - Obturator nerve or branches thereof;

- Shoulder Joint -Suprascapular nerve or branches thereof (most common for shoulder pain);

- Elbow Joint -Radial nerve and Ulnar nerve or branches thereof; and

- Finger Joints-Superficial radial nerve; or combinations thereof.

The nerve may be located in any tissue. Any suitable nerve or nerve fibres may be targeted for pain relief and/or muscle/motor neuron inhibition and/or modulation of function. In one embodiment, the targeted nerve may be a peripheral nerve. The nerve may be a sensory neuron. In addition, any suitable muscle may be targeted for modulation of function, for example cardiomyocytes.

The peptide drug may be administered by injection, with an injection volume of about 1 - 10ml or 1-5ml in humans, for example when administered to the intraarticular space of the knee joint. Additionally, or alternatively the peptide drug may be administered at a dose in the range 1 mg/ml - 30mg/ml of polymer and covalently bound and/ or mixed cationic peptides. In one embodiment, the peptide drug is administered at a dose of about 1 microg/ml to 1500 microg/ml of cationic peptides

**Other Aspects**

According to another aspect of the invention, there is provided a method of manufacturing the peptide drug according to the invention, the method comprising the step of covalently bonding cationic peptides to the polymer and forming a gel, colloid, or solution in a carrier.

According to another aspect of the present invention, there is provided a method of treatment of a subject for pain or a motor neuron disorder selected from spasticity, migraine, dystonia or essential tremor, the method comprising the local administration of a peptide drug to a subject at the site of the pain or motor neuron disorder, wherein the peptide drug comprises a polymer in a carrier, wherein the polymer comprises a plurality of cationic peptides covalently bound to the polymer and/or cationic peptides mixed with the polymer.

In one embodiment, the pain is any pain associated with the peripheral nervous system (i.e. not the central nervous system). In one embodiment, the pain may a pain from neuropathic pain, nociceptive pain, painful bladder syndrome, cystitis, or vaginal atrophy. The pain may be joint pain, such as from osteoarthritis, or tissue pain, for example from tissue damage, disease, or infection.

The treatment may be localised treatment, for example by injection at the site of the pain or motor neuron to be modulated. Migraine pain may be treated by localised injection into the forehead and/or temple of a subject.

According to another aspect of the present invention, there is provided the use of the peptide drug according to the invention for pain relief or motor neuron modulation in a subject.

According to another aspect of the present invention, there is provided the use of the peptide drug according to the invention for modulation of nerve cell activity, cardiomyocyte or muscle cell electrical activity in a subject. The subject may be afflicted with a disorder associated with nerve cell activity, cardiomyocyte or muscle cell electrical activity.

According to another aspect of the present invention, there is provided the use of the peptide drug according to the invention for treatment of conditions related to abnormal activity of muscle cells - for example cardiac arrhythmias by blockade of inappropriate pacemaker cell activity.

The term "abnormal activity of muscle cells" may include an activity causing a pathology, such as irregular or excessive contraction of muscle cells, such as in arrhythmia.

The use may be for a cosmetic procedure, for example to relax facial skeletal muscles.

According to another aspect of the present invention, there is provided a kit comprising the peptide drug according to the invention, and a syringe.

The peptide drug may be pre-loaded in the syringe. The kit may further comprise instructions for local administration of the peptide drug to a site of pain, such as a joint, or a site for motor neuron blockade/ modulation.

**Cationic Peptide Dose Treatment**

According to another aspect of the invention, there is provided the use of cationic peptides, such as Cationic Arginine-Rich Peptides (CARPs), for the localised modulation of nerve cell activity, cardiomyocyte or muscle cell electrical activity in a subject, wherein the treatment comprises the administration of the cationic peptides to the subject by localised injection into a tissue or joint at the site of the pain and/or at the site of a motor neuron condition and/or the site of the muscle cells, and optionally wherein the cationic peptides are administered at a dose of 1- 10ml and about 1 microg/ml to 1500 microg/ml of cationic peptides.

The cationic peptides are administered at a dose of 1 - 5ml and about 1 microg/ml to 1500 microg/ml of cationic peptides. The administration may be local administration.

In one embodiment, the cationic peptides comprise or consist of CARPs.

Arginine based peptides have been observed to modulate TRPV1 channels (pain sensing channel; part of the vanilloid receptor (VR) family). However, other channels, such as sodium channels and/or calcium channels and/or potassium channels, may be additionally or alternatively blocked by such peptides. VR modulators maybe be used as analgesics. Arginine based peptides have been shown to be effective as VR modulators at specific concentrations, however, at higher concentrations these peptides have proven to be toxic *in-vitro.* Advantageously, the present invention establishes a concentration that is safe and effective for poly-arginine solution to block neuron activity without impacting viability. Here we have shown that arginine based peptides formulated in salt buffers can act as injectable treatment for osteoarthritic joints. Arginine based peptides can also be used to modulate VR receptors and/ or sodium and/or calcium and/or potassium ion transport across nerve cell membranes and the membrane of related excitable cells.

**Cartilage Regeneration**

According to another aspect of the invention, there is provided the use of the peptide drug according to the invention herein or cationic peptides, such as Cationic Arginine-Rich Peptides (CARPs), for the regeneration of cartilage tissue and/or the prevention of cartilage degeneration and/or protection (i.e. ability to perform normal function despite the local presence of pathology or inflammatory mediators) of cartilage producing cells (chondrocytes).

According to another aspect of the invention, there is provided the use of the peptide drug according to the invention herein or cationic peptides, such as Cationic Arginine-Rich Peptides (CARPs), for the regeneration of cartilage tissue component, such as collagen II, and/or the prevention of collagen II degeneration from cartilage.

According to another aspect of the invention, there is provided the use of the peptide drug according to the invention herein or cationic peptides, such as Cationic Arginine-Rich Peptides (CARPs), for the treatment of osteoarthritis.

According to another aspect of the invention, there is provided a method of treatment or prevention of osteoarthritis, the method comprising the local administration of the peptide drug according to the invention herein or cationic peptides, such as Cationic Arginine-Rich Peptides (CARPs) into a joint.

According to another aspect of the invention, there is provided a method of enhancement of chondrocyte activity for cartilage regeneration or maintenance, the method comprising administering the peptide drug according to the invention herein or cationic peptides, such as Cationic Arginine-Rich Peptides (CARPs) to chondrocytes, optionally wherein the chondrocytes are in vivo, such as in a joint.

The cartilage may be in a joint, such as a knee joint. The peptide drug according to the invention herein or cationic peptides, may be administered locally, such as into the joint.

Advantageously, the peptide drug according to the invention has been shown both *in-vitro* and *in-vivo* to offer regeneration in osteoarthritis and chondroprotection. The treatment may comprise the enhancement of chondrocyte activity for cartilage regeneration or maintenance.

In one embodiment the peptide drug according to the invention herein or cationic peptides, such as Cationic Arginine-Rich Peptides (CARPs), are used to enhance or maintain collagen II production, for example from chondrocytes. The use may be *in vitro* or *in vivo.*

## Definitions

In one embodiment, modulation of nerve cells includes inhibition of the action potential generation or excitation of the action potential generation.

In one embodiment, modulation of muscle cell electrical activity includes inhibition or excitation. Preferably the modulation is inhibition.

In one embodiment, modulation of cardiomyocytes includes inhibition or excitation. Preferably the modulation is inhibition.

A "gel" is understood to be a semi-solid matrix of polymers, which exhibits no flow when in the steady-state, although the continuous phase may still diffuse through this matrix.

The term "colloid" is understood to mean a dispersion of polymer and peptides in a carrier. The colloid is understood to be liquid in form.

The term "solution" is understood to mean the polymer and/or peptides are dissolved in a solvent carrier.

The term "biocompatible" is understood to include non-toxic to the human or animal body. To be biocompatible, the peptide drug may not cause an immune response.

The term "biodegradeable" is understood to include the ability to breakdown over time in the tissue or body of a human or animal, and/or in the environment. The time for complete degradation may be at least 1 week, at least 1 month, at least 2 months, at least 6 months, or at least 12 months. The time for complete degradation may be no more than 12 months. The time for complete degradation may be no more than 6 months.

The term "localised", "local delivery", local administration" or "localised treatment" or similar, is intended to refer to the administration of treatment directly to a site of the pain or site of relevant pathology, for example by injection into the effected tissue, or directly into the afflicted joint. For delivery into a joint, the peptide drug may be administered into a joint space, such as into the synovial fluid. The treatment may not be administered systemically.

A "covalent bond", also called a molecular bond, is a chemical bond involves the sharing of electron pairs between atoms. These electron pairs are known as shared pairs or bonding pairs, and the stable balance of attractive and repulsive forces between atoms, when they share electrons, is known as covalent bonding.

The term "inhibition" used herein may be a total or partial inhibition of activity, such as channel activity or electrical activity.

The skilled person will understand that optional features of one embodiment or aspect of the invention may be applicable, where appropriate, to other embodiments or aspects of the invention.

Embodiments of the invention will now be described in more detail, by way of example only, with reference to the accompanying drawings.

## Figures

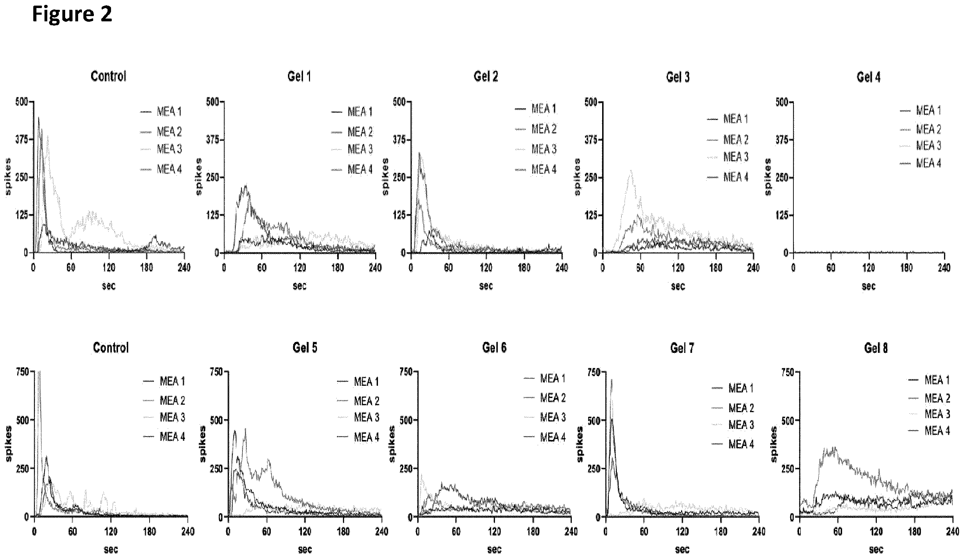

**List of tested materials and example embodiments of the invention with reference to appropriate figure. (See also Fig. 2).**

| Material | Description | Test Data | Concentration Tested | Figure # |
|---|---|---|---|---|
| Hyaluronic Acid | 2.2-2.4 MDa | Patch Clamp (DRG)<br>MEA (DRG)<br>Viability<br>Viscosity<br>*in-vivo* (MIA/ vF)<br>*in-vivo* (Acute/ vF) | 400μg/ml<br>2 mg/ml<br>2.5 mg/ml<br>10-20 mg/ml<br>10 mg/ml<br>400 μg/ml | 1A-C<br>3C<br>4A-B<br>5A<br>6B<br>8A-B |
| Hyaluronic Acid | 0.75 MDa | Viscosity | 10-20 mg/ml | 5A |
| PEG | 20 kDa | Patch Clamp (cortical neuron) | 400 μg/ml | 10D |
| Hydrobloc P01 | HA 2.2-2.4 MDa covalently bound to PLA 15 - 70 kDa (1 : 0.05 gram : gram ratio) | Patch Clamp (DRG)<br>MEA (DRG)<br>Viability<br>Viscosity<br>Sizing<br>*in-vivo* (MIA/ vF)<br>*in-vivo* (Acute/ vF)<br>Stability<br>Regeneration | 400μg/ml<br>400μg/ml<br>2.5 mg/ml<br>10-20 mg/ml<br>N/A<br>10 mg/ml<br>400 μg/ml<br>2.5 mg<br>200 μg/ml | 1A-C<br>3A<br>4A-B<br>5A<br>5C<br>6B<br>8A-B<br>9<br>12A |
| Hydrobloc C04 | HA 2.2-2.4 MDa mixed with PLA 15-70 kDa (1 : 0.05 gram : gram ratio) | Patch Clamp (DRG)<br>MEA (DRG)<br>Viability | 400 μg/ml<br>400 μg/ml<br>2.5 mg/ml | 1A-C<br>3A<br>4A-B |
| Hydrobloc P02 | HA 2.2-2.4 MDa covalently bound to PLA 9.6 kDa (1 : 0.011 gram : gram ratio) | MEA (DRG)<br>Viability<br>Viscosity<br>*in-vivo* (MIA/ vF) | 400 μg/ml<br>2.5 mg/ml<br>10-20 mg/ml<br>10 mg/ml | 3A<br>4A-B<br>5A<br>6B |
| Hydrobloc P03 | HA 2.2-2.4 MDa covalently bound to PLA 1.9 kDa (1: 0.002 gram : gram ratio) | MEA (DRG)<br>Viability<br>Viscosity<br>FTIR<br>Sizing<br>*in-vivo* (MIA/ vF)<br>*in-vivo* (MIA/ WB)<br>Cardiomyocytes | 400 μg/ml<br>2.5 mg/ml<br>10-20 mg/ml<br>N/A<br>N/A<br>10 mg/ml<br>13 - 20 mg/ml<br>2 mg/ml | 3A<br>4A-B<br>5A<br>5B<br>5C<br>6B<br>7A<br>11B |
| Hydrobloc P03.1 | HA 2.2-2.4 MDa covalently bound to PLA 1.9 kDa (1: 0.001 gram : gram ratio) | MEA (DRG)<br>Viscosity<br>FTIR<br>Sizing<br>*in-vivo* (MIA/ WB) | 2 mg/ml<br>10-20 mg/ml<br>N/A<br>N/A<br>20 mg/ml | 3C<br>5A<br>5B<br>5C<br>7A |
| Hydrobloc P03.3 | HA 2.2-2.4 MDa covalently bound to PLA 1.9 kDa (1: 0.004 gram : gram ratio) | Viscosity<br>FTIR<br>Sizing<br>*in-vivo* (MIA/ WB)<br>Regeneration | 10-20 mg/ml<br>N/A<br>N/A<br>13 - 20 mg/ml<br>20 mg/ml | 5A<br>5B<br>5C<br>7A-B<br>12B |

(continued)

| Material | Description | Test Data | Concentration Tested | Figure # |
|---|---|---|---|---|
| Hydrobloc P03.12 | HA 2.2-2.4 MDa covalently bound to PLA 1.9 kDa (1: 0.05 gram: gram ratio) | Viscosity | 10-20 mg/ml | 5A |
| Hydrobloc P03.11 | 0.75 MDa covalently bound to PLA 15 - 70 kDa (1: 0.05 gram : gram ratio) | Sizing | N/A | 5C |
| Hydrobloc P03.13 | HA 0.75MDa covalently bound to PLA 1.9 kDa (1: 0.004 gram : gram ratio) | Viscosity | 10-20 mg/ml | 5A |
| Hydrobloc B10-PEG | PEG 20 kDa covalently bound to PLA 1.9 kDa (1 : 0.002 gram : gram ratio) | Patch Clamp (cortical neuron) Patch Clamp (motor neuron) | 1 mg/ml 1 mg/ml | 10B 11A |
| Poly-Arginine only | 15 - 70 kDa | Viability | 0.125 mg/ml | 4A-B |
| | 9.6 kDa | Viability | 0.0275 mg/ml | 4A-B |
| | 1.9 kDa | MEA | 10 $\mu$g/ml - 1000 $\mu$g/ml | 3B |
| | | Viability | 0.005 mg/ml | 4A-B |
| | | Ca$^{2+}$ Imaging (cortical neuron) | 1 $\mu$g/ml | 10A |
| | | Patch Clamp (cortical neuron) | 1 $\mu$g/ml | 10C |

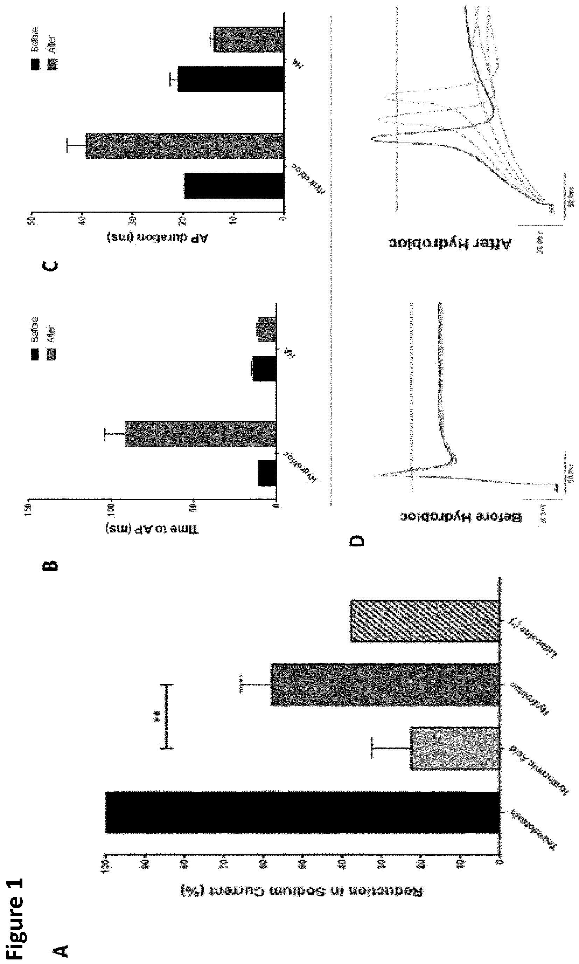

**Figure 1:** Sodium current flow as assessed using patch clamp on dorsal root ganglia (DRG) (sensory) neurons treated with 400 $\mu$g/ml Hydrobloc P01 or Hyaluronic Acid (HA). **Panel A (n=15):** Percentage reduction of sodium current. TTX inhibits current by approximately 99% acting as a control. Hydrobloc reduced sodium current by 57.9% while HA reduced sodium current by 22.6% (p = 0.0022). Lidocaine is reported in the literature to reduce sodium current by 38%. Data were presented as mean $\pm$ SEM. **Panel B (n = 15):** Hydrobloc treated DRG cells showed an increase in the time between action potentials (i.e., a reduction in action potential frequency) and **Panel C (n = 15):** an increased action potential duration when compared to DRGs treated with HA and no treatment controls (HA having no effect with compared to control). **Panel D:** In summary, Hydrobloc causes a general suppression of the events associated with action potentials, with consequent reduced pain signal transmission by the nerve. A similar effect was observed for the PEG-PLA prototypes.

**Figure 2:** Effect of various peptide drug embodiments on electrical activity of DRG cells using Microelectrode Array (MEA). n = 4 arrays used per sample, with samples diluted 1:10 from stock concentration in all cases. Each MEA has a population of DRGs and records the collective electrical activity. Cells are stimulated with capsaicin which causes a transient increase in activity from approximately 0 - 120 s (Control). Peptide drug treated DRGs show a general suppression of electrical activity compared to control despite the presence of capsaicin. See notes in table below for other important results and conclusions.

| Gel # | Composition Concentration *gram : gram ratio (polymer : peptide)* | Embodiment Name | Notes |
|---|---|---|---|
| Gel 1 | **HA bound to Lys monomer** 20 mg/ml *1:0.050* | Hydrobloc P08 | Monomer causes suppression of activity |
| Gel 2 | **HA bound to PLA (9.6 kDa)** 20 mg/ml | Hydrobloc P02 | |

(continued)

| Gel # | Composition<br>Concentration<br>*gram : gram ratio*<br>*(polymer : peptide)* | Embodiment Name | Notes |
|---|---|---|---|
| | 1 : 0.011 | | |
| Gel 3 | **HA bound to Arg monomer**<br>20 mg/ml;<br>*1:0.050* | Hydrobloc P04 | Monomer causes suppression of activity |
| Gel 4 | **PLA (15-70 kDa)**<br>1 mg/ml<br>*N/A* | Hydrobloc C01 | Free large (42.5 kDa average) PLA cytotoxic. |
| Gel 5 | **HA bound to PLA (15 - 70 kDa)**<br>20 mg/ml<br>*1:0.050* | Hydrobloc P01 | Binding to HA suppresses activity, and removed toxicity (compare to Gel 4) |
| Gel 6 | **HA bound to PLA (1.9 kDa)**<br>20 mg/ml<br>*1 :0.002* | Hydrobloc P03 | Best overall suppression of activity. |
| Gel 7 | **HA bound to PLL (15-30 kDa)**<br>20 mg/ml<br>*1 :0.027* | Hydrobloc P05 | |
| Gel 8 | **HA mixed with PLA (15 - 70 kDa)**<br>20 mg/ml<br>*1:0.050* | Hydrobloc C011 | Mixing also causes suppression of activity (compare to Gel 5). |

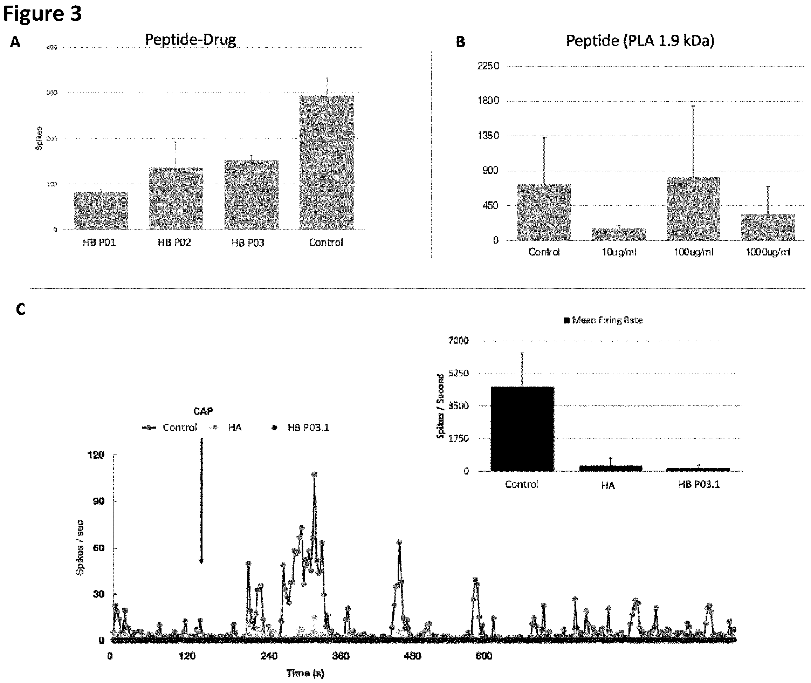

**Figure 3:** Effect of various peptide drug embodiments and peptide alone on electrical activity of DRG cells using Microelectrode Array (MEA). n = 3 arrays used per sample. Each MEA has a population of DRGs and records the collective electrical activity. Cells are stimulated with capsaicin (control) which causes a transient increase in activity from approximately 0 - 120 s (Control). Action potentials (spikes) can be recorded and summed over the time course of the experiment. **Panel A:** Peptide drug treated DRGs show a general suppression of electrical activity compared to control despite the presence of capsaicin as seen by lower total spike count. 400 $\mu$g/ml used in all cases. **Panel B:** PLA 1.9 kDa a sample cationic peptide (and is the peptide bound to HA in HB P03) shows a dose range effect on modulation of DRG activity as seen be different spike count compared to control at different concentrations. Most suppression is seen at 10 $\mu$g/ml which compares favourably to the effect seen when covalently bound as HB P03. **Panel C:** Peptide drug (HB P03.1) compared to HA and control with results shown as the time course of spikes recorded and mean activity. HB P03.1 seen to reduce spikes by approximately half compared to HA alone. 2 mg/ml used in all cases.

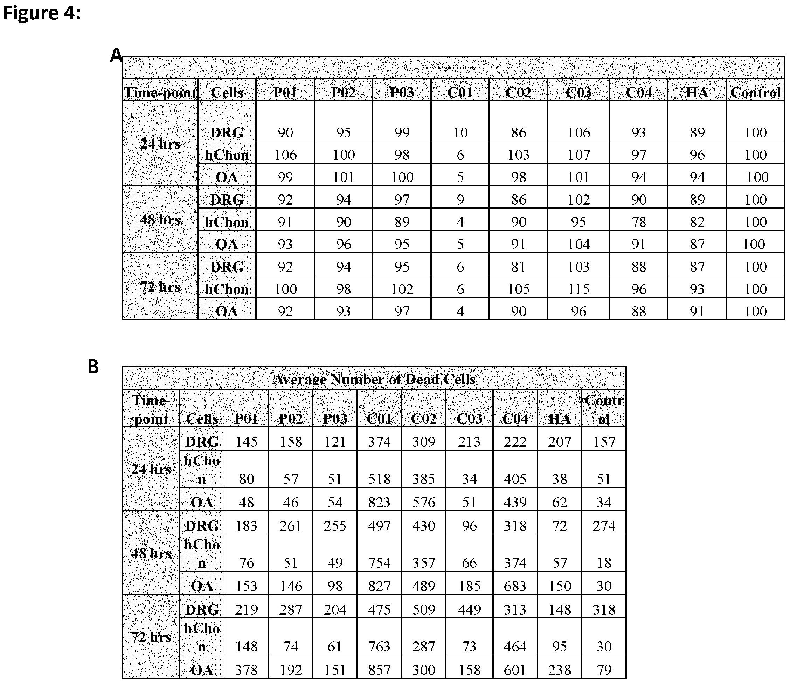

**Figure 4:** Data of the effect of various peptide drug embodiments and peptide alone on metabolic activity and viability of cell types (DRG: dorsal root ganglion; hChon: human normal chondrocytes; OA: human OA chondrocytes). n = 3 in all cases; all tested at 2.5 mg/ml of HA or equivalent using gram: gram ratio (See Table). Details on test materials given in below table. **Panel A:** Metabolic activity assayed using Alamar Blue test - No significant difference in peptide drug embodiments compared to control at 24-72 hours in three different cell lines providing evidence that peptide drug does not affect cellular metabolic activity. Longer chain CARPS (e.g., C01) do appear to negatively affect metabolic activity but not when covalently bound (P01) or mixed (C04) with HA. **Panel B:** Viability assessed using Live/ Dead assay. No significant difference between peptide drug at 24 - 72 hours in DRGs compared to control. Longer chain CARPS (e.g., C01) do appear to negatively affect viability but not when covalently bound (P01) or mixed (C04) with HA. Smaller CARPS (C03) have best viability profile, again improved when bound to HA (P03).

| Embodiment Name | Composition<br>Concentration<br>*gram : gram ratio (polymer : peptide) <if applicable>* |
|---|---|
| Hydrobloc P01 | **HA bound to PLA (15 - 70 kDa)**<br>2.5 mg/ml<br>*1:0.050* |
| Hydrobloc P02 | **HA bound to PLA (9.6 kDa)**<br>2.5 mg/ml<br>*1 : 0.011* |
| Hydrobloc P03 | **HA bound to PLA (1.9 kDa)**<br>2.5 mg/ml<br>*1 :0.002* |
| Hydrobloc C01 | **PLA (15-70 kDa)**<br>0.125 mg/ml |
| Hydrobloc C02 | **PLA (9.6 kDa)**<br>0.0275 mg/ml |
| Hydrobloc C03 | **PLA (1.9 kDa)**<br>0.005 mg/ml |
| Hydrobloc C04 | **HA mixed with PLA (15 - 70 kDa)**<br>2.5 mg/ml<br>*1:0.050* |
| HA | **HA 2.2 - 2.4 MDa**<br>2.5 mg/ml |

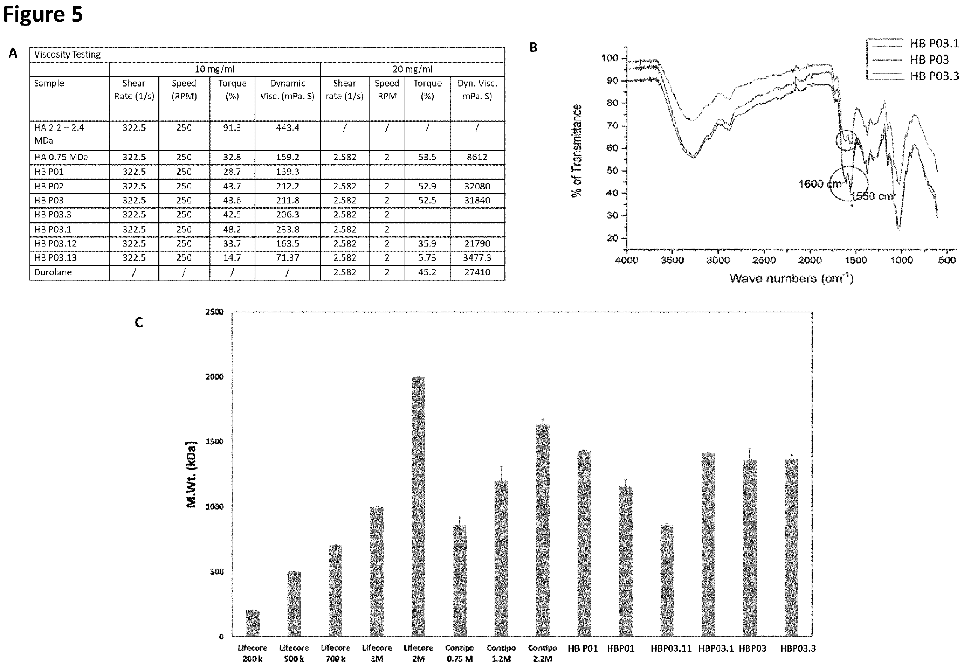

**Figure 5:** Physicochemical characterisation of various peptide drug embodiments. **Panel A:** Viscosity results indicate that prototypes decrease in viscosity compared to HA (2.2-2.4 MDa) at the same concentration. Viscosity is found to increase with concentration and be dependent on the chain. When the concentration was increased to 20 mg/mL. Hydrobloc prototypes appear to show greater viscosity than similar products (e.g, Durolane®) already found on the market for the treatment of osteoarthritis (OA) pain, but of the same order of magnitude. At 20 mg/mL hyaluronic acid becomes too viscous for viscosity determination at 2 RPM. **Panel B:** FT-IR Spectra of Hydrobloc prototypes. Here the splitting of the peak at 1600 cm$^{-1}$ into two separate peaks demonstrates the successful bonding of PLA to HA. **Panel C:** Sizing using HPLC of Hydrobloc prototypes using Lifecore and Contripro HA standards as reference. All prototypes shown here used were found similar in size to the Contipro 2.2 MDa HA standard as expected, with HB P03.11 similar to the 0.75 MDa as expected. In general, the prototypes were smaller than the corresponding HA - evidence of PLA crosslinking and compacting the size of the molecule.

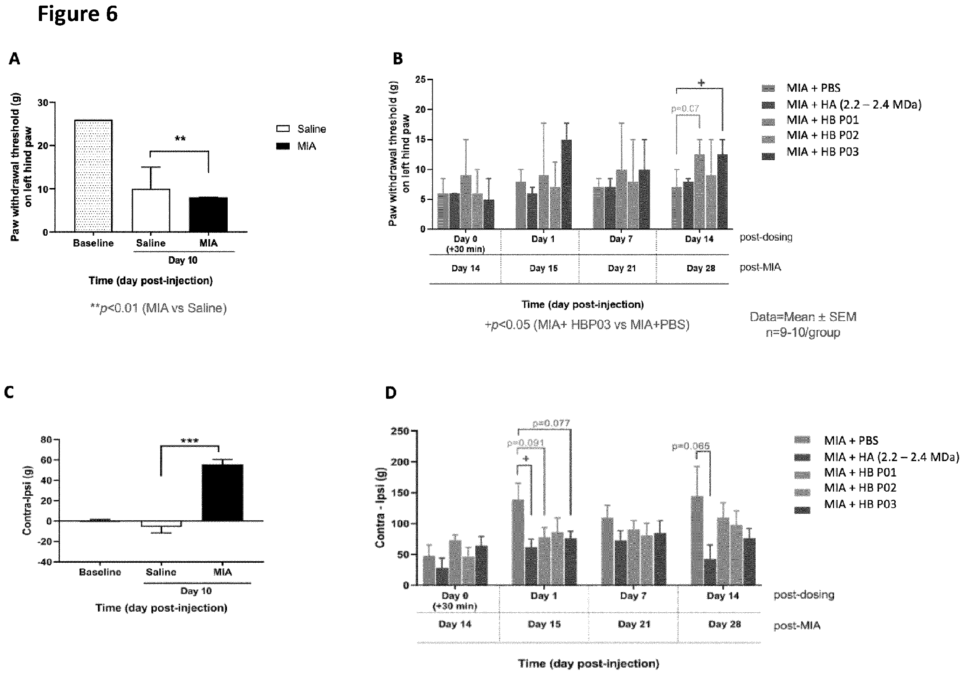

**Figure 6:** Effects of vehicle (phosphate buffered saline (PBS)), HA (2.2 - 2.4 MDa), and various peptide drug embodiments (HB P01, HB P02, HB P03) on pain behavioural tests in the dedicated mono-iodo acetate (MIA) model of knee osteoarthritis. MIA when injected into the joint causes progressive degradation of cartilage with loss of joint structure and function, effectively modelling the human disease. The model takes approximately 10 days to develop. All materials tested at 10 mg/ml, left knee joint as index joint. **Panel A:** MIA injected into the knee joint of rats causes a drop in the paw withdrawal threshold as a measure of pain when tested using the von Frey (vF) test when compared to saline injection. The effect is progressive and pronounced from Day 10 on. **Panel B:** Rats received a single injection of the assigned treatment in the left knee joint on Day 14 post-MIA and assessed in the vF test 30 min later and thereafter on Days 15, 21, and 28. HA did not affect PWT in MIA-injected rats. Treatment with HB P03 increased PWT in MIA rats on Day 28 compared with vehicle-treated MIA rats. HB P01 produced a strong trend in increasing PWT in the MIA rats on Day 28 ($p=0.072$, vs MIA+PBS). In comparison, PWT were similar in MIA rats treated with B03P02 and vehicle. Data is expressed as median with interquartile range and analysed using Kruskal Wallis test followed by post hoc Mann-Whitney U-test, where appropriate (n=10 per group). $+p<0.05$ (MIA+ HB P03 vs MIA+PBS).

**Panel C:** MIA injected into the knee joint of rats causes a weight bearing asymmetry between the hindlegs. Data (mean ± SEM) is analysed as the difference in weight borne between intact (right) and injured (left) hind paws. Effect of saline or MIA injection into the left knee joint. MIA-injected rats showed deficit in weight bearing in the left hind limb on Day 10 post-injection (p<0.001, t-test; saline: n=10, MIA: n=49). Rats received a single injection of the assigned treatment in the left knee joint on Day 14. **Panel D:** Effects of vehicle, HA, and peptide drug embodiments (HB P01, HB P02, HB P03) on weight bearing asymmetry in MIA-injected rats. HA reduced MIA-induced weight bearing asymmetry. Treatment with Hydrobloc prototypes HB P01 (p=0.091, vs MIA+PBS) and HB P03 (p=0.077, vs MIA+PBS) produced strong trends in decreasing MIA-induced weight bearing deficit on Day 15 but not at later time points. MIA effect was analysed using Student's t-test. Treatment effect was analysed using repeated measures ANOVA followed by post hoc Dunnett's test, where appropriate (n=9-10 per group). ***p<0.001 (MIA vs Saline); +p<0.05 (MIA+HA vs MIA+PBS).

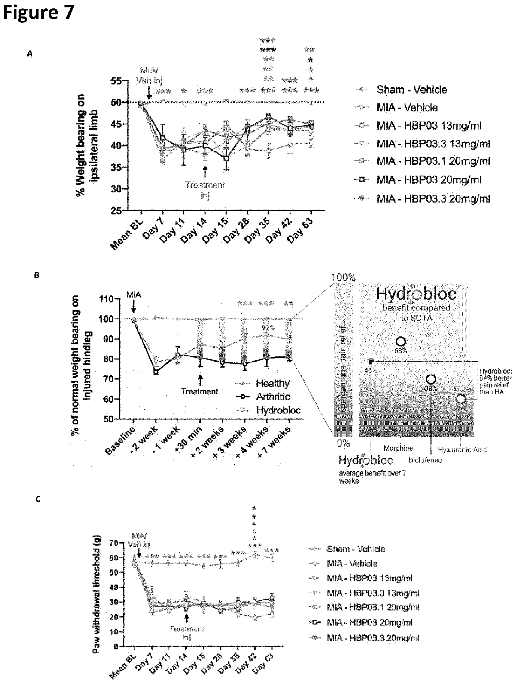

**Figure 7:** Effects of vehicle (phosphate buffered saline (PBS)) and various peptide drug embodiments (HB P03, HB P03.1, HB P03.3) on injured (left) hind paw of rats in the dedicated mono-iodo acetate (MIA) model of knee osteoarthritis as assessed using two behavioural tests of pain. MIA when injected into the joint causes progressive degradation of cartilage with loss of joint structure and function, effectively modelling the human disease. The model takes approximately 10 days to develop. **Panel A:** Weight bearing (WB) test: MIA injected into the knee joint of rats causes a drop in the percentage weight borne on the injured hindlimb (usually 50% of body weight in the test) as a measure of pain when tested using the WB test. The effect is progressive and pronounced from Day 10 on: Rats received a single injection of the assigned treatment in the left knee joint on Day 14 post-MIA and assessed in the vF test 30 min later and thereafter on Days 15, 21, 28, 35, 42, and 63. Multiple Hydrobloc prototypes show significant pain-relieving effects at multiple time points compared to arthritic control (MIA - Vehicle). This demonstrates long-lasting pain relief. Concentration effects and trends in level of cationic peptide binding are also noted (with HB P03.3 20 mg/ml the best performing). (* p < 0.05, ** p < 0.01, *** p < 0.001). **Panel B:** Results of HB P03.3 20 mg/ml focused on WB result and compared to healthy and arthritic controls. At peak effectiveness Hydrobloc gives 92% pain relief (i.e., 92% of normal weight bearing restored). The recovery band is defined as the interval between the healthy and arthritic controls with Hydrobloc giving on average 46% pain relief over 7 weeks using this band. This compares favourably to state-of-the-art analgesics. In particular HB P03.3 is 84% more effective than commercial HA product Artz® under similar testing conditions. Data is expressed as mean with SEM and analysed using Repeated Measures ANOVA test followed by post hoc Dunnettest (MIA-vehicle as control group), where appropriate (n=5-6 per group). **Panel C:** Pain assessed as the paw withdrawal threshold (g) in the ipsilateral hind paws using the von Frey test. MIA reduced PWT in the ipsilateral hind paw (mechanical hypersensitivity) from Day 7. MIA-injected rats that were treated with Hydrobloc prototypes displayed significant improvements in ipsilateral PWT relative to the MIA-Vehicle control group on Day 42. Data are presented as mean ± SEM and analysed using repeated measures ANOVA followed by post hoc Dunnett's test (MIA-Vehicle as control group). *p<0.05, *** p < 0.001 (given treatment group versus MIA-vehicle); n = 4-6/group.

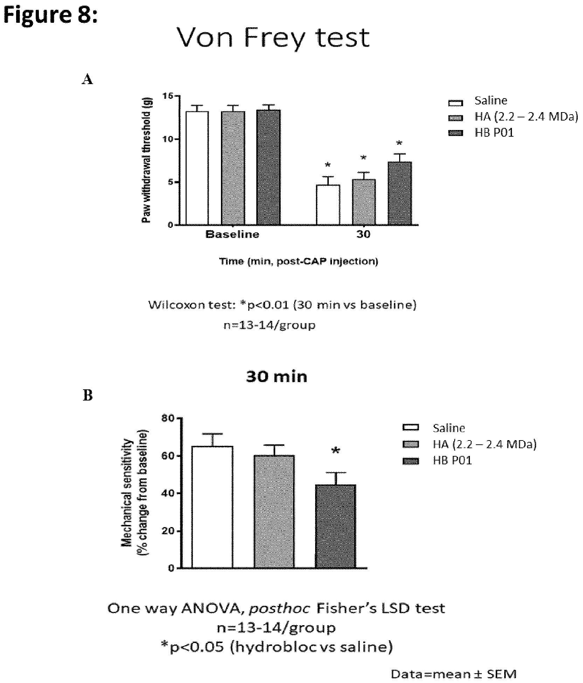

**Figure 8:** Effects of peptide drug embodiment (HB P01) on von Frey threshold in an acute capsaicin model of pain. **Panel A:** The baseline von Frey Threshold is identical for all three groups before administration of capsaicin and one of saline, HA (400 μg/ml), or HB P01 (400 μg/ml). At 30 minutes after administration, the von Frey Threshold is significantly reduced in all groups indicating capsaicin has induced allodynia. The reduction in Von Frey Threshold is significantly less in the Hydrobloc HB P01 group compared to either saline or HA. **Panel B:** The reduction in von Frey Threshold at 30-minutes as a percentage of baseline. While saline and HA show a ~ 60% decrease in tolerance to pain (i.e. von Frey threshold decrease of ~ 60% compared to baseline), the Hydrobloc HB P01 group has a significantly lower ~ 40% decrease in the von Frey threshold despite the presence of capsaicin. This indicates an analgesic effect of Hydrobloc in acute pain.

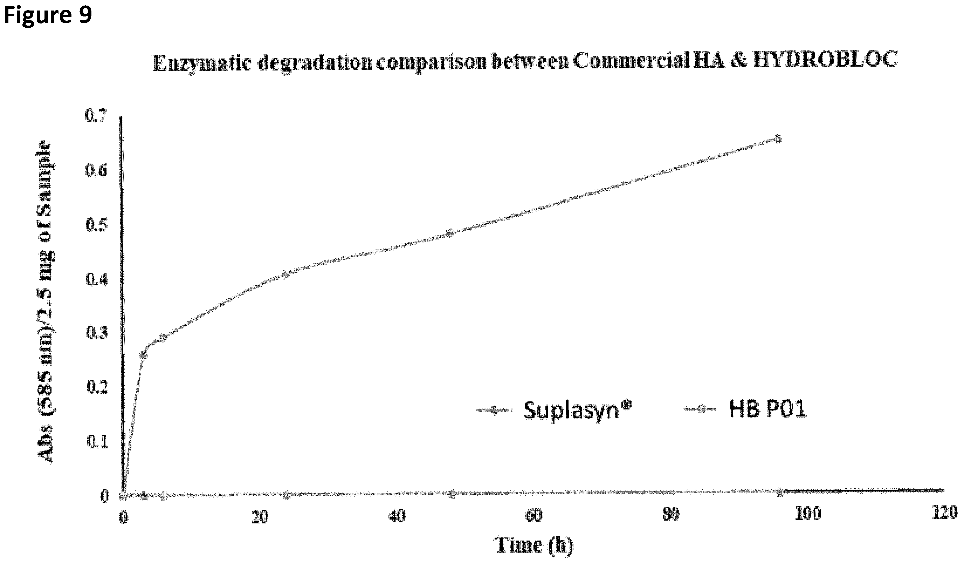

**Figure 9:** A key value proposition of Hydrobloc is a long-lasting duration of action of over 6-months. Hydrobloc samples underwent an FDA approved accelerated stability study. Hydrobloc HB P01 was shown to be significantly more stable than Suplasyn® (commercial HA) when exposed to hyaluronidase in an accelerated study providing evidence of a long-lasting effect, and the benefits of covalently binding the peptide to the polymer to increase stability.

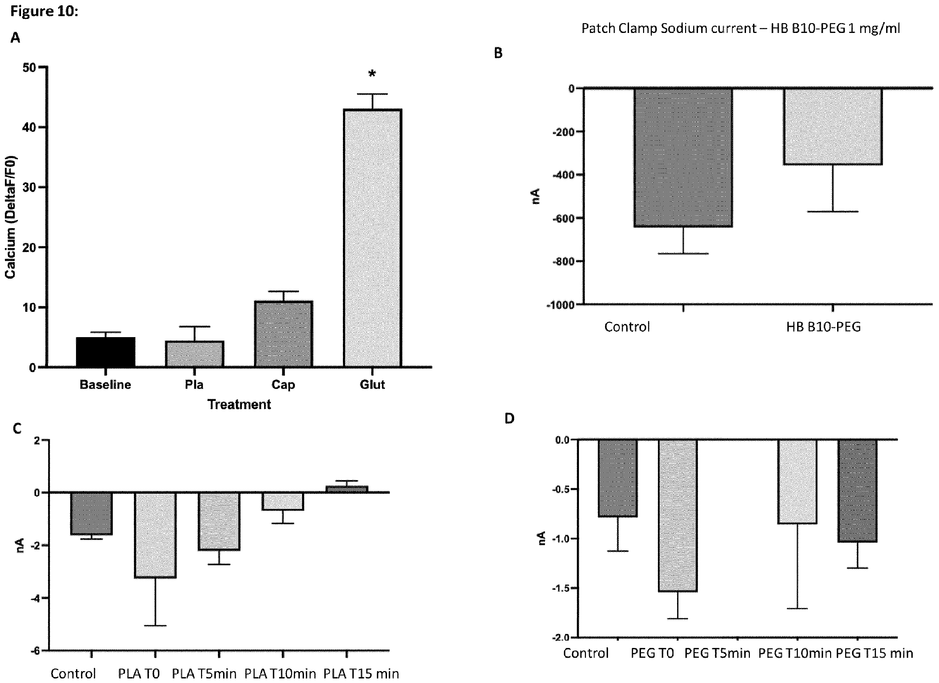

**Figure 10:** Testing suggests cationic peptides act on the TRPV1 pathway or related pathways associated with response to painful stimuli in neurons. TRPV1 channels on neurons open in response to stimuli like capsaicin. Once open cations like calcium flow into the cell, which triggers action potentials and the sensation of pain. Cells tested were cortical neurons, n = 3 unless noted. **Panel A:** Calcium imaging is used to measure calcium flow across neural cell membranes. Cells treated in sequence with no chemical (control), PLA (1.9 kDa at 1 μg/ml), capsaicin, and then glutamic acid. Neural cells treated with cationic peptide and then capsaicin do not show an increase in electrical activity. This indicates the cationic peptide blocks the capsaicin (TRPV1 pathway response). Exposure to glutamic acid does stimulate the cell despite the presence of cationic peptide indicating the glutamate response is unaffected and hence specificity for the TRPV1 or related pathways.

**Panel B:** The suppression of the capsaicin response and evidence of an effect on the TRPV1 pathway is also seen when the cationic peptides are covalently bound or mixed with polymer as shown in Figure 2 and here with HB B10-PEG (PEG covalently bound with PLA 1.9 kDa). **Panel C:** Patch clamp recording of the sodium current (downstream from TRPV1 channels) show the cationic peptide block (PLA 1.9 kDa at 1 μg/ml) to have a time dependent effect - getting stronger with time. This is an effect seen by TRPV1 agonists that are used as analgesics such as capsaicin. **Panel D:** Polymer alone (e.g., PEG at 400 μg/ml) fails to show suppression of sodium current indicating the need for the presence of cationic peptide.

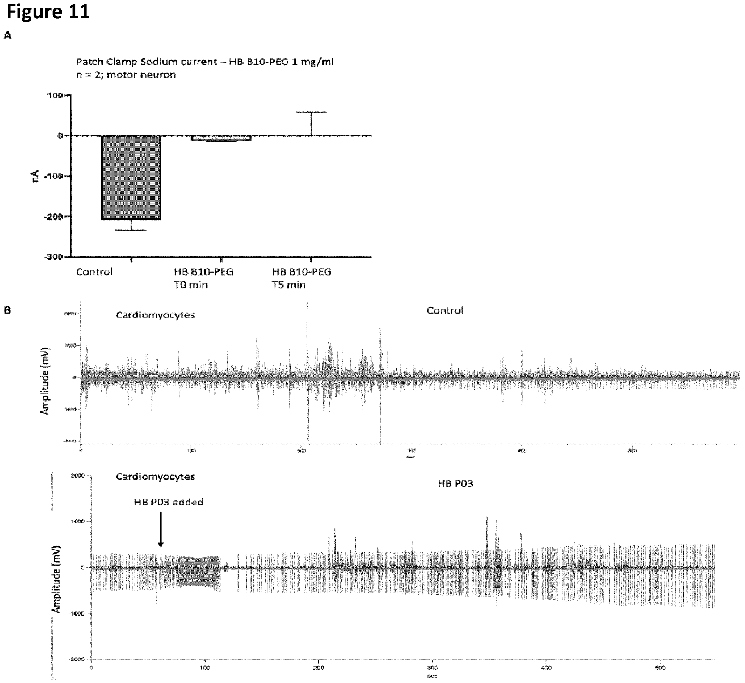

**Figure 11:** Peptide drug embodiments have also been shown to modulate the activity of excitable tissues such as motor neurons and cardiomyocytes. **Panel A:** HB B10-PEG (PEG covalently bound with PLA 1.9 kDa) at 1 mg/ml shown to block motor neurons as assessed using patch clamp analysis of sodium current, and in a time dependent manner similar to as shown in Fig. 10C. **Panel B:** HB P03 (2 mg/ml) when added to a culture of cardiomyocytes is seen to modulate the pattern of electrical activity with an overall negative chronotropic and positive inotropic effect seen (i.e. slower but stronger pattern of contractions) compared to control.

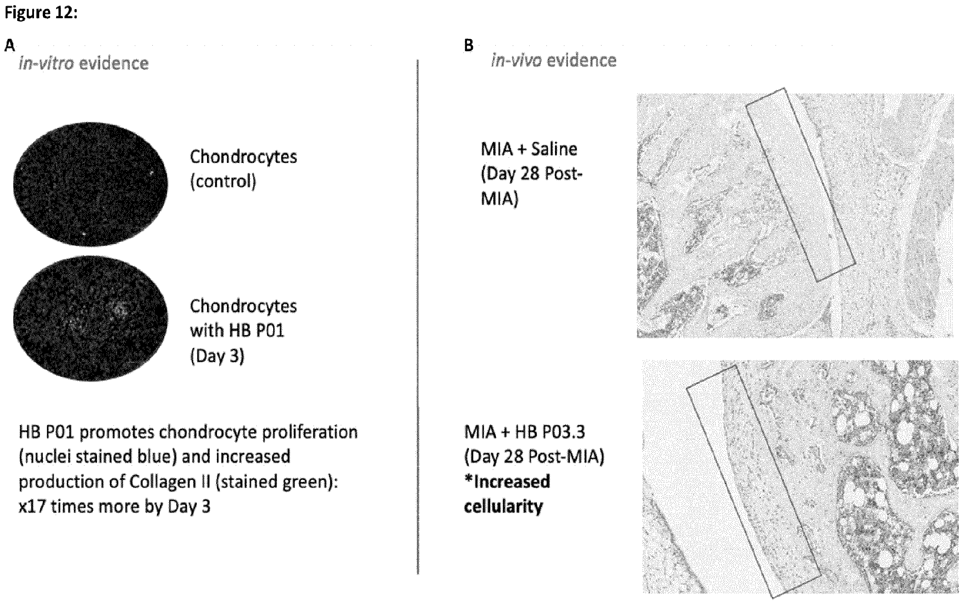

**Figure 12:** Peptide drug embodiments have also been shown to offer regeneration in osteoarthritis and chondroprotection. CARPs are reported in the literature to reduce inflammation associated with osteoarthritis and protect against cartilage degeneration. These effects have been seen in Hydrobloc prototypes both *in-vitro* and *in-vivo*. **Panel A:** Chondrocytes (cartilage producing cells) produce collagen II as part of the cartilage matrix. Chondrocyte cells treated with HB P01 (200 μg/ml) produced x17 the amount of collagen II in 72 hours compared to control. As collagen II is a component of cartilage this is evidence of enhancement of chondrocyte activity and cartilage regeneration, both of which would be desirable in osteoarthritis treatments. (Collagen II stained as light dots in image, chondrocytes as darker dots). **Panel B:** Histology of knee joints of rats sacrificed 28 days after treatment with MIA. MIA degrades cartilage and is used to induce osteoarthritis in these animal models. The cartilage zone is highlighted by a rectangle. Control animals' cartilage is seen to be largely acellular indicating chondrocyte loss. Animals treated with HB P03.3 (20 mg/ml) seen to have an increased cellularity in the cartilage zone indicative of chondrocytes being present and active.

## Examples

One aspect of the present disclosure comprises taking a medically approved biodegradable polymer, such as hyaluronic acid or a salt thereof, chemically modified to have properties that modulate the activity of excitable cells such as neurons. One method to achieve this is to attach a nerve blocking functional group to polymer chains forming, or comprised in, the biodegradable polymer. One such functional group that is reported to block nerve activity is guanidine. It is well reported in the literature that the mechanism in which neurotoxin such as tetrodotoxin blocks nerve activity is through the positively charged guanidine group present in the molecule. Alternatively, the full neurotoxin molecule may be attached to the polymer chains of the biodegradable polymer, where the neurotoxin molecule is covalently bonded to the biodegradable polymer. It is also well reported in the literature that neurotoxins block nerve activity and can be used to treat pain. However, in the treatment of pain, neurotoxins do not give long lasting effects due to fact that the neurotoxin can easily migrate away from a target site.

The peptide drug can contain positively-charged ionic channel-binding functional groups, which are attracted to the negatively charged carboxylate groups in ion-channel openings on a nerve surface. In clinical use, the peptide drug or gel may be injected into the target area of the nerve, and may act to block the sodium ion channels of the nerve cell, thereby preventing the entry of sodium ions into the intracellular space. Since the flow of sodium ions into nerve cells is a necessary step for nerve conduction, the peptide drug or gel will reduce, optionally effectively block, all pain signals from the nerve. Other channels exist that may act as a target and would give a similar overall effect on the nerve or excitable tissue, such as the TRPV1 channel.

Chemical modification of the biodegradable polymer to attach nerve blocking molecules can be achieved by applying any of many well-known methods in the art, including the following surface chemistry methods.

The biodegradable polymer to which the nerve blocking functional groups/molecules are covalently linked are designed to ensure that they can diffuse to the surface of a nerve where the ion channels are present. Suitable designs are based, for example, on the size of the polymer chains. The polymer chains of the biodegradable polymer can be designed to be any size ranging from 1-1000 million Daltons. Control to polymerisation chain lengths in polymer reactions is well documented in the scientific literature. For example, hyaluronic acid polymer chain length can be controlled by polymerisation chemistry methods such as using microwave to control reaction conditions. These methods are extensively documented in, for example, Larrañeta et al., 2018 (Carbohydrate Polymers 2018 Feb 1; 181: 1194-1205).

## Degradation of the biodegradable polymer *in-vivo*

The biodegradable polymer can be designed to degrade *in-vivo* from 6 month to 5 years. Degradation can be controlled and tailored to ensure the required degradation times are achieved. The main factors that determine the degradation process are the chemical structure of the polymer chain in the polymer backbone; hydrophilic/hydrophobic groups; polymer morphology; molecular weight; surface area; catalysts; and additives of resorbable polymers. The chemical structure of the polymer chain in the polymer backbone is the most important parameter. Generally, anhydrides degrade faster than the ester group, which in turn degrades faster than the amide group. Based on this, it is possible to predict degradation of a given polymer. The hydrophilic/hydrophobic character is another important parameter in polymer degradation. Poly(glycolic acid) degrades faster than the more hydrophobic poly(lactic acid), although they have the same degradable chain in the polymer backbone. The degradation rate is further influenced by polymer morphology. Crosslinkers may be used to slow down the degradation profile of hyaluronic acid. The crosslinkers bind HA polymer chains to each other, creating a polymer 'network' and transforming the viscous liquid into a gel. Crosslinking HA polymer chains transforms the HA solution into a gel. Crosslinker molecules bind individual HA polymer chains to create a network, which manifests macroscopically as a gel mass. This gel is a single unit, imposing a physical and chemical barrier to enzymatic and free radical breakdown. Because the gel network is multiply connected, enzymes and free radicals can break down the chains only in much smaller portions at a time. Moreover, due to their large size, enzymes can have difficulty penetrating the gel network, which will in effect contribute to a slower degradation. This translates into longer persistence of the gel of the present invention at a target site *in vivo*. Common crosslinkers include 1,4-butanediol diglycidal ether (BDDE), and di-vinyl sulfone (DVS). Both react with hydroxyl sites on the HA chains and offer similar results in slowing down enzymatic and free radical degradation of HA gels once injected. BDDE may be used as a crosslinker, binding together two HA polymer chains. BDDE (1,4-butanediol diglycidal ether) crosslinking agent may be used to bind HA polymer chains to each other, transforming liquid HA solutions into gels. Both the primary hydroxyl site (-CH2OH) and secondary hydroxyl sites (-CHOH) within the HA monomeric unit are possible target sites for reaction with BDDE.

**Crosslinking of Hyaluronic acid**

Materials: Hyaluronic acid sodium salt (HA, also called hyaluronan or sodium hyaluronate) with an average molecular weight of 1.5 106 Da, was supplied by Shangdong Freda Biopharm Co., Ltd. (Jinan, China) as dry powders. 1,4-Butanediol diglycidyl ether (BDDE) was purchased from Sigma Chemical (St. Louis, MO, USA). Unless otherwise specified, all other chemical reagents used were supplied by Sinopharm Chemical Reagent, of analytical grade and used as received without further purification.

Cross-linking of Hyaluronic acid (HA): HA was first dissolved in 1% NaOH at a concentration of 10 wt%, after which BDDE was added to the HA solution with vigorous stirring. The final concentrations of BDDE were 0.4 vol%, 0.6 vol%, 0.8 vol% and 1 vol%, respectively. The solution was then allowed to crosslink at 40 °C for 5 h, followed by being dried at room temperature for 3 days. Phosphate buffered saline (PBS: NaCl, 9 mg mL-1 ; $KH_2PO_4$, 0.03 mg mL-1; $Na_2HPO_4$ $2H_2O$, 0.14 mg mL-1 ; pH 7.0) of 500 mL was then added to the above crosslinked HA to make it swell, after which it was put into dialysis bag and dialyzed sequentially with excessive deionized (DI) water and PBS to remove the residual BDDE. The resulting gel was adjusted with PBS to obtain a gel with a HA concentration of 20 mg mL-1 and then smashed with a homogenizer to obtain gel particles of 0- 400 mm. Before being used, the obtained injectable gel was sterilized in a high-pressure steam sterilizer set at 120 °C, 20 min.

When the biodegradable polymer is hyaluronic acid, the hyaluronic acid used for covalent linking with the sodium ion channel blocker can be un-crosslinked, or can be crosslinked, optionally as disclosed above.

**Curing of gel *in situ***

The gel may be designed to be cured in situ. This gives the added benefit to ensure the gel does not migrate from the target site and would also give the gel additional mechanical strength. It is difficult to inject very viscous materials; however, a low viscosity polymer solution could be injected to the target nerve site and by curing in situ the gel can cross-link and form a strong solid support of high mechanical strength. Curing in situ would be achieved by using a chemical crosslinker and injecting the polymer gel in two more parts. For example, parts A & B may be crosslinked using UV light or any method which promotes polymer cross-linking to form a solid polymer matrix in vivo.

**Targeting the gel to different nerve fibres or excitable cells**

The gel may be formulated to selectively target different nerve fibres or other excitable cells. This can be achieved by a concentration effect or by increasing the volume. For example, C-type pain fibres are non-myelinated and of 0.2-1.5 micron diameter while A-delta pain fibres are myelinated and of 1 - 5 micron diameter. These pain fibres are of smaller diameter to afferent or motor fibres. This differing anatomy may offer the opportunity for a different pattern of ligands or gel to target pain fibres and not interfere with the other nerve types, thereby reducing potential adverse effects.

**Delivery of the gel to target site**

The gel may be delivered to the site of action (the vicinity of the target nerve) using a needle attached to a syringe loaded with the gel. A range of needles are available depending on the intended site of action / viscosity of the gel and other factors. In order to facilitate delivery of small amounts of gel in a relatively wide area encompassing the target nerve, the needle can have a mechanism that delivers aliquots of gel as a series of micro-injections. Initially, the clinician delivers the needle to a central area at which point a mechanism is engaged that causes the needle tip to partially retract/ move/ inject over a specified automated cycle to result in optimal widespread delivery of the gel.

The syringe could have a second chamber with a counteracting agent to neurotoxin or other nerve blocking ligand attached to the gel. This counteracting agent could be a direct antagonist of the toxin (for example, 4-aminopyridine may be used as an antidote to tetrodotoxin) or may cleave the group linking the ligand to the gel, thus de-anchoring the gel. Such a mechanism to counteract the neurotoxin/ nerve blocking ligand on the gel would be desirable in the event of excessive amount of the gel being delivered or incorrect initial placement. The counteracting agent could also be delivered in a separate syringe delivered by a similar or modified injecting pattern if there were unacceptable adverse effects experienced by the patient in the period after initial treatment.

Optionally, the counteracting agent may be one that causes direct break-up of the gel itself, for example, a chemical that lyses polymer cross-links. Such as the following: bacterial β-endoglycosidases, bacterial β-exogtycosidase, eukaryotic enzymes: β-Endogtycosidases, β-Exoglycosidases, and/or peptioglycan polymer of β-(1-4)-N-Acetyl-D-glucosamine units.

The skilled person will appreciate that different grades of gel with differing types and amount of neurotoxins/ nerve blocking ligands can be designed for different target sites and for larger/ smaller nerves. For example, the trigeminal nerve branches have quite distinct dermatomes meaning targeting of a given branch should allow targeting of effect to the respective dermatome. More extensive targeting may require delivery of neurotoxin/ nerve blocking ligand gel to the trigeminal ganglion for widespread effect, or alternatively separate targeting of each branch.

**Materials and Methods**

**Materials**

Hyaluronic Acid mol wt 2.2-2.4 MDa) was purchased from Contripro (Czech Republic), N-(3-Dimethylaminopropyl)-N'-ethylcarbodiimide hydrochloride (EDC) (E1769), N-Hydroxysuccinimide (NHS) (130672), Poly-L-arginine hydrochloride mol wt 15,000-70,000 Da (P7762), Hyaluronidase from Streptomyces Hyalurolyticus (H1136), MES hemisodium salt (M0164) and Dialysis tubing cellulose membrane, (molecular weight cut-off = 14,000, D9527) from Sigma-Aldrich (Ireland), All other chemicals, cell culture media and reagents were purchased from Sigma-Aldrich (Ireland) unless otherwise stated.

Peptide drug synthesis was done by carbodiimide coupling reaction between HA and PA (polyarginine) in MES buffer solution.

Specifically, HA was dissolved overnight at room temperature at 1% w/v in 0.1 M MES buffer (pH 6.5).

To activate the carboxyl groups of the HA, NHS followed by EDC were added drop-wise to the HA solution at a HA: EDC: NHS mole ratio of 1:50:25 and stirred with a magnetic stirrer for 1 hr at 37° C. Next PA (used for guanidine functionalisation of HA) was added to the HA solution at a HA:PA mole ratio of 1:20. The reaction was then allowed to proceed by stirring at 700 rpm for 24 h at 37° C to obtain the final product of PA functionalized HA (PAFHA; i.e. peptide drug A).

After completion of the stirring, the solution was dialysed (MWCO 14 kDa) against deionised water for 5 days with 3-4 water changes per day and lyophilised for further analysis.

The resultant HA-PA polymer (peptide drug A) was suspended in Phosphate Buffered Saline (PBS) forming a gel of concentration 400 μg/ml with an 18% degree of crosslinking. The grafting of PA onto HA was confirmed using gold standard characterisation techniques: Fourier-Transform Infrared Spectroscopy (FTIR) and Zeta-Potential measurements. FTIR showed the amide peak present on the peptide drug A (see Figure 3 herein), while Zeta-Potential showed the overall charge of the peptide drug A had shifted considerably from negative (-40 mV) towards positive (-1 mV). Both results verify success of the synthesis pathway, which is shown with the chemical structure of peptide drug A in Figure 2.

Details of chemicals used provided in Table 1.

**Table 1:** Chemicals used in the synthesis of peptide drug A of the invention.

| Chemical Name | Abbreviated Name | Molecular weight (Da) | Other Relevant Physiochemical Characteristics | Company |
|---|---|---|---|---|
| Sodium Hyaluronate | HA | 2.2 - 2.4 MDa | Dry Powder | Contipro A.S. Czech Republic |
| 2-(N-morpholino)ethanesulfonic acid | MES | 206.73 | Powder | Sigma-Aldrich |

(continued)

| Chemical Name | Abbreviated Name | Molecular weight (Da) | Other Relevant Physiochemical Characteristics | Company |
|---|---|---|---|---|
| N-hydroxysuccinimide | NHS | 115.09 | Powder | Sigma-Aldrich |
| 1-ethyl-3-(3-dimethylaminopropyl)-carbodiimide hydrochloride | EDC | 191.7 | Powder | Sigma-Aldrich |
| Poly-L-Arginine | PA | 15,000-70,000 Da | Powder | Sigma-Aldrich |
| Spectrum™ Spectra/Por™ Biotech Cellulose Ester (CE) Dialysis Membrane Tubing | CE membrane | MWCO 100 kDa | Cellulose Ester tube | Fisher Scientific |

**Example 1- Physiochemical characterisation**

This details the characterisation of the final product, summarised in Table 2 - peptide drug A.

*General characterisation*

*Zeta-sizer*

Size analysis detected that peptide drug A ("Prototype") had an average size of 38 nm ($\pm$ 4 nm).

*Zeta potential*

A zeta-potential analysis of peptide drug A was performed as follows:
Zeta potential (ZP or $\zeta$-potential) analyses were performed using Zetasizere instrument (Malvern) with three replicates. The ZP measurements were performed at 25°C under the Smoluchowski approximation method and the results were expressed as average values and standard deviation (S.D.) for three replicates (n = 3).

*Fourier transform infrared spectroscopy (FTIR)*

Fourier Transform Infrared Spectroscopy (FTIR) of peptide drug A was performed as follows:
The analysis of the functional groups present in HA, PA and peptide drug A were made by a Fourier transform infrared spectrophotometer (Alpha, Bruker, Germany). All spectra were recorded at the resolution of 4 cm$^{-1}$ in the range of 400-4000 cm$^{-1}$.

*Degradation*

Peptide drug A samples were degraded using Streptomyces derived hyaluronidase in an accelerated enzymatic study. The commercial HA product Suplasyn® (Commerical HA) was used as a control.
The measurement of the released N-acetyl glucosamine (NAG) from peptide drug A was performed according to the Reissig et al. method [Reissig JL, Strominger JL, Leloir LF. A modified colorimetric method for the estimation of N-acetylamino sugars. J Biol Chem 1955;217:959-96].
Briefly, a diluted 1:10 Ehrlich's reagent (Sigma Aldrich) in acetic acid was added to the tubes. The samples were vortexed and incubated for 20 min at 37°C, to develop a violet colour, proportional to the released NAG content in each sample. The tubes were centrifuged at 1000 g for 15min to remove the gel fragments and the turbidity in the reaction. Then, the samples were read with a microplate reader at 585 nm wavelength against the blank prepared with the only phosphate buffer and the Ehrlich's reagent.

**Table 2:** Peptide drug A batch classification.

| Zeta-Potential | FTIR Performed | Degradation Study Notes |
|---|---|---|
| Shift from -40 mV (HA) to -15 mV (B00P01) | Figure 3 | Stable to *Streptomyces* hyaluronidase for at least 96 hours in accelerated test (Fig. 4) |

## Results

### General characterisation

### Zeta-sizer

Size analysis of the gel prototype gave a reading of 38 nm ($\pm$ 4 nm)

### Zeta potential

The zeta-potential study showed the overall charge shifted from negative (-40 mV) in the starter HA material had towards positive (-15 mV) in the peptide drug A product (Table 2). This might be due to the increase of primary amine groups in the peptide drug A structure.

### Fourier transform infrared spectroscopy (FTIR)

**Figure 5B** depicts the FTIR spectra of HA and sample peptide drugs. A broad stretching band of hydroxyl groups was recorded at around 3350 cm$^{-1}$ in the FTIR spectrum of HA. Two bands at 1650 and 1550 cm$^{-1}$ corresponded to carbonyl stretching bands of carboxylic acid and amide, respectively. Ether bands were assigned at around 1151 and 1030 cm$^{-1}$ (PAVIA, D. L.; LAMPMAN, G. M.; KRIZ, G. S. Introdução àespectroscopia. 4. ed. São Paulo: Cengage Learning, 2010.700 p) for HA and HA-PA spectra. A band at around 1400 cm$^{-1}$, which was due to the -COOH group in HA.
In the peptide drug A spectrum, the intensity of the bands at around 1650 and 1550 cm$^{-1}$ (due to amide I and amide II) was increased due to the formation of the amide linkage in peptide drug A. One new shoulder peak was also observed at around 3100 cm$^{-1}$ in the peptide drug A spectrum which was a fermi resonance of N-H stretch with the overtone of the amide II band. Whereas the intensity of the peak generated for free -COOH group at around 1400 cm$^{-1}$ was reduced in the peptide drug A spectrum compared with HA due to its involvement in the EDC-NHS coupling reaction for the formation of a amide linkage between HA and PA. Considering all these findings, the succeeded synthesis of peptide drug A could be proved.

### Degradation

Peptide drug A samples were degraded using Streptomyces derived hyaluronidase in an accelerated enzymatic study. The commercial HA product Suplasyn® (Commerical HA) was used as a control. Rapid degradation was observed for commercial HA whereas there was no degradation was observed in peptide drug A for the first 96 hrs (see **Figure 9**).

### Conclusion

All of the findings prove the successful synthesis of peptide drug A and the rate of enzymatic degradation is very slow in peptide drug A compared to commercial HA.

### Example 2 - *In vitro* assessment

### Materials and methods

### Materials

Calcium and magnesium were purchased from Invitrogen (U.S). All chemical, cell culture media and reagents were purchased from Sigma-Aldrich (Ireland) unless otherwise stated.

**Cell culture**

Dorsal Root Ganglia (DRG) sensory neurons were extracted from adult rats (Cúram). The DRG explants were dissected from the cervical and thoracic regions of the spine of neonatal rat pups (P5) and placed in cold HBSS (1x) without $Ca^{2+}$ / $Mg^{2+}$ (Invitrogen, USA). After cleaning the remnants of peripheral nerve processes, DRGS were seeded in 12-well glass-coated laminin plates. Cells were feed every 2/3 days and they were processed for single cell patch clamping.

**Electrophysiology: Single cell patch clamp**

Whole cell patch clamp configuration was used to record from 100 day iPSC derived neurons and DRG neurons. All recordings were performed in warm extracellular bath solution, with 140 mM NaCl (Sigma-Aldrich 71387), 5 mM KCl (Sigma-Aldrich P9333), 2 mM $CaCl_2$ (Sigma-Aldrich C5670), 2 mM $MgCl_2$ (Sigma-Aldrich M8266), 10 mM HEPES sodium salt (Sigma-Aldrich H7006) and 10 mM glucose at pH 7.4. Images were taken under Zeiss Axiovert 200 (40X).

Patch pipettes were pulled on borosilicate glass capillaries (Harvard apparatus GC150TF-7.5) using a Zeitz DMZ puller (Werner Zeitz, Germany). The glass pipettes were filled with intracellular solution (123 mM $C_6H_{11}KO_7$, 10 mM KCL, 1 mM $MgCl_2$, 10 mM HEPES potassium salt (Sigma-Aldrich H0527), 1 mM EGTA, 0.1 mM $CaCl_2$, 1.5 mM ATP magnesium salt (Sigma-Aldrich A9187), 0.2 mM GTP sodium salt hydrate (Sigma-Aldrich 51120) and 4 mM glucose with resistance of 4.0-5.5 M$\Omega$.

Recordings were made using the EPC10 patch clamp amplifier from HEKA. Voltage-dependent $Na^+$ and $K^+$ currents were recorded in voltage clamp mode from a holding potential of -70 mV; voltage step depolarization was applied up to +20 mV in 10 mV increments and recorded for 200 ms. All data were recorded unfiltered for voltage and current clamp recording at 50.0 kHz and 20.0 kHz, respectively.

Intrinsic firing properties of neurons were recorded in current clamp mode, with step current injections from a holding value of -5 pA up to +45 pA, for 500 ms, in 10 pA increments. Spontaneous action potential (AP) was recorded in current clamp mode at zero current injection (resting membrane potential) just after whole cell configuration was achieved. Data analysis was performed using patchmaster and fitmaster (HEKA).

**Statistics**

All data were expressed as mean $\pm$ SEM. All data were tested for normality using Shapiro-Wilk normality test. Statistical analysis was performed using student t-test with a $p < 0.05$.

**Results**

**Figure 1:** Peptide drug A shows a significant alteration in $Na^+$ current. **(Figure 1D)** Representative image of single cell patch recording. Representative traces of voltage clamp recording from Before an addition of peptide drug A and from After the addition of peptide drug A on DRG neurons, showing fast inward $Na^+$ currents followed by slow outward $K^+$ currents, in response to different voltage step applied. **(Figure 1A)** 400 $\mu$g/ml of peptide drug A significantly reduced inward sodium current in comparison to 400 $\mu$g/ml of Hyaluronic acid. Data were presented as mean $\pm$ SEM.

The majority (96%) of patched cells responded in a voltage-dependent manner, and their currents increased in line with the voltage step applied (-70 mV to +20 mV). Cells were patched at resting membrane potential of 34 $\pm$ 7.5 in voltage clamp mode. 180 ul of peptide drug A or Hyaluronic acid were added to the bath solution at the final concentration of 400 $\mu$g/ml. The sodium and potassium currents were measured at different time points (3, 5, 7, 15, 30 minutes). The inward $Na^+$ currents were significantly decreased relative to Hyaluronic acid (Hyaluronic acid 22.61 $\pm$ 9.8; peptide drug A 58 $\pm$ 7.5, p= 0.008) (Figure 5B-D).

**Figure 1B and C:** Peptide drug A significantly alters action potential characteristics. 400 $\mu$g/ml of peptide drug A significantly changed the action potential duration while there was no change after the addition of 400 $\mu$g/ml of Hyaluronic acid. The time to action potential threshold from the site of current injection is significantly longer in peptide drug A in comparison to Hyaluronic acid. Data were presented as mean $\pm$ SEM.

The effects of Hyaluronic acid and peptide drug A in voltage-gated $Na^+$ and $K^+$ currents were subsequently investigated in evoked AP kinetics. The AP duration were longer and time to the AP threshold were significantly longer after the addition of peptide drug A (denoted as "Prototype") compared to HA **(Figure 1B,C).**

**Conclusion**

During pain the neurons becomes hyper-excitable and spontaneous firing develop at the injury site in addition of dorsal root ganglion cell bodies. This hyperexcitability partly occurs due to the accumulation of sodium channels.

Blocking sodium channels can reduce excitability and neuronal firing and hence reduction in pain sensation.

These results show that there is a reduction of inward $Na^+$ current after the addition of peptide drug A. Reduction in $Na^+$ also reduced action potential duration and the time to the action potential generation. These results can suggest a reduction in hyperexcitability of neurons and hence sensation of pain.

### *Example 3 - In vivo* assessment

### Materials and methods

### Materials

Details on the chemicals, cell culture media and reagents referred to in this Example are provided in Tables 1 and 2 if used in previous Examples, and otherwise in Table 4.

Details on the treatments used is provided in Table 3.

### Animal

All the animals used in this study were healthy, male Sprague Dawley rats from Charles River Laboratories (U.S). The rats were acclimatised for a period of not less than a week. During this acclimatisation period no prophylactic or therapeutic treatment was administered. The animals were group housed with no more than two rats per cage (size dependent). The housing was a ventilated cage rack system, under a 12 hour light/ dark cycle (07:00 - 19:00), and under suitable humidity and temperature conditions. The rats were given water ad libitum, and a standard rodent diet

### *In-vivo study design*

A Capsaicin with Mechanical Allodynia study was performed, with von Frey filaments used to quantify the degree of mechanical allodynia. The animal procedures were conducted according to Institutional Animal Care and Use Committee (IUCAC) guidelines, with for example only healthy animals were placed in the study. Full details of the study are provided in Protocol A below and summarised in Table 5.

The rats were allowed acclimatise to the test room, and to the testing chamber. On the day prior to the study proper, the rats were tested for baseline response using von Frey filaments.

At the start of the study animals were randomised to treatment groups, with distribution according to baseline von Frey test results. N = 36 rats were used in total, allocated into three groups as shown in Table 5. The rats were X weeks of age, 36 rats (mean body weight 250-300 grams) at the start of the study. In addition, the study was blinded. The agents were administered by intraplantar injection into one hind paw. Allodynia was induced with 25 $\mu$l (30 $\mu$g) of capsaicin. At a time point 15 minutes later 25 $\mu$L of each test agent was administered, with the nature of the test agent as given in Table 3. Following test agent administration, von Frey testing was performed at 6 time points (0 min, 30 min, 90 min, 3 h, 6 h, and 24 h) with n = 4 animals tested from each group at a given time point. On completion of the study the animals were sacrificed by transcardial perfusion with 4% paraformaldehyde. The treated paws of n = 4 per group were collected for histological analysis.

Table 3: Treatments Used in the *in vivo* study

| Treatment Name | Solute | Solvent | Notes | Final Concentration |
|---|---|---|---|---|
| Vehicle | N/A | PBS (See Table 4) | | N/A |
| TA1 | HA | PBS (See Table 4) | | 400 $\mu$g/ ml |
| TA2 | Peptide drug A | PBS (See Table 4) | | 400 $\mu$g/ ml |

Table 4: Details of Chemicals Used *in vivo* Test (if not already listed previously)

| Chemical Name | Abbreviated Name | Constituents | Company | Batch Number | Date Opened | Expiry Date |
|---|---|---|---|---|---|---|
| Phosphate Buffered Saline | PBS | | | | | |

(continued)

| Chemical Name | Abbreviated Name | Constituents | Company | Batch Number | Date Opened | Expiry Date |
|---|---|---|---|---|---|---|
| Capsaicin | | | Sigma Aldrich | | | |

Table 5: *in vivo* Study Design: Capsaicin with Mechanical Allodynia

| Group # | Treatment | Group Size | Days of Dosing | Dose | Route | Evaluation/ Endpoints |
|---|---|---|---|---|---|---|
| 1 | Vehicle | 12 | 1 | 0 | Intraplantar | von Frey - At 0, 30, 90, 3, 6 h and 24 h (n = 4 / group; 12 total at 24 h) post capsaicin* |
| 2 | TA1 | 12 | 1 | 25 μL of 400 μg/ mL | | |
| 3 | TA2 | 12 | 1 | 25 μL of 400 μg/ mL | | von Frey withdrawal threshold |
| | | | | | | Perfusion and paw collection (n = 4/ group; 12 total) |

*Animals will be observed for spontaneous nocifensive behavior in the 1st 10 minutes following capsaicin injection, and again for 5 minutes before the 90, 3 & 6 h capsaicin time points.

**Results**

**Behavioural analysis: Capsaicin Mechanical Allodynia (von Frey)**

Summary *of in vivo* von Frey test results for Peptide drug A. The baseline von Frey Threshold is identical for all three groups before administration of capsaicin and one of saline, HA, or peptide drug A. At 30 minutes after administration, the von Frey Threshold is significantly reduced in all groups indicating capsaicin has induced allodynia. The reduction in Von Frey Threshold is significantly less in the peptide drug A group compared to either saline or HA. **Figure 8A:** The reduction in von Frey Threshold at 30-minutes as a percentage of baseline. While saline and HA show a ~ 60% decrease in tolerance to pain (i.e. von Frey threshold decrease of ~ 60% compared to baseline), the peptide drug A group has a significantly lower ~ 40% decrease in the von Frey threshold despite the presence of capsaicin.

The behavioural test results are summarised in **Figure 8B.** There was a statistically significant higher von Frey Threshold (i.e. analgesic effect) 30-minutes after administration of capsaicin with peptide drug A of the present invention (7.41 grams), compared to HA (5.35 grams) and saline (5.31 grams) ($p < 0.05$ One way ANOVA).

**Conclusions**

The results from this *in vivo* study show that peptide drug A demonstrated a clear analgesic effect evidenced by the higher von Frey threshold compared to control - see **Figure 8.** A statistically higher von Frey threshold was shown by peptide drug A (7.41 g) with respect to that of HA (5.35 g) or saline (5.31g). These results translated into a ~ 40% reduction in von Frey threshold relative to baseline despite the presence of capsaicin for peptide drug A, and a ~ 60% reduction for both HA and saline. These results imply that the general dampening of action potential activity as shown in Figures 6 and 8 may translate into a dampening of afferent pain sensing neuron activity in this whole animal model.

Finally, the peptide drug A was judged by the investigators to be easy to administer to the paw by intraplantar injection. The administration was not felt to be any more difficult than either of the other test agents. Although this study was designed to assess efficacy, these reports lend evidence as to the device having flow characteristics supporting ease of device delivery.

**Protocols**

Protocol A: Capsaicin with Mechanical Allodynia (von Frey) Methodology

On the day of the study, rats were placed in the testing room to acclimate for 30 minutes, and then placed into the testing chamber for 30-60 minutes prior to testing with von Frey filaments. The animals may also have received additional chamber acclimation days prior to baseline testing;

- Testing chambers were composed of wire bottom cubicles with opaque Plexiglas walls, allowing access to the underside of their paws;

- Rats were tested once for baseline response the day prior to dosing and testing;

- Rats were divided into treatment groups by the scatter arrangement based on the baseline;

After baseline determination, rats were administered test agents into the plantar surface of one hind paw followed by capsaicin (pre-treatment time 15 minutes prior to capsaicin) and tested at the time points listed in Table 5 above:

1. For induction of allodynia, rats were injected with 25 $\mu$l (30 $\mu$g) of capsaicin into the plantar surface of one hind paw to induce acute, persistent, or chronic inflammatory pain. The needle was inserted through the toes, directed towards the heel and advanced to the middle of the plantar surface. A successful injection was noted by the formation of a small bleb.

2. At several time points post capsaicin mechanical allodynia was measured with von Frey filaments utilising the up-and-down method, or the simplified up-and-down method.

3. Allodynia was tested by perpendicularly touching the plantar surface of the animal's ipsilateral hind paw with von Frey filaments (0.4 - 15g) causing slight buckling of the filament for approximately 5 seconds. Based on the response pattern and the force of the final filament, the paw withdrawal threshold (g) was calculated.

### Example 4 - *In vivo* assessment

Example 4 tested the efficacy and safety of different prototypes, and at different doses in the rat MIA model of OA.

### Materials and Methods

Details on the prototypes, chemicals, induction agents, and treatments referred to in this Example are provided in Tables 6, 7, 8, and 9 respectively.

**Table 6:** Composition of prototypes tested in the Example 4 *in vivo* study.

| Prototype Code: | HA | PLA |
|---|---|---|
| B09P3.3 | MW: 2200-2400 kDa 1g (0.434 $\mu$mol) | MW: 1.9 kDa 4.4 mg (2.352 $\mu$mol) |
| B09P3.2 | MW: 2200-2400 kDa 1g (0.434 $\mu$mol) | MW: 1.9 kDa 2.2 mg (1.176 $\mu$mol) |
| B09P3.1 | MW: 2200-2400 kDa 1g (0.434 $\mu$mol) | MW: 1.9 kDa 1.1 mg (0.588 $\mu$mol) |

**Table 7:** Chemicals used in the Example 4 *in vivo* study.

| Chemical Name | Abbreviated Name | Constituents | Company | Batch Number |
|---|---|---|---|---|
| Phosphate Buffered Saline | PBS | Sodium chloride 9mg; Potassium Dihydrogen Phosphate 0.03mg; Disodium Hydrogen Phosphate Dihydrate (Merck) 0.14mg; Water for Injection q.s. 1ml | Sigma Aldrich (Merck) unless stated | N/A |
| Sodium monoiodoacetate | MIA | Sodium iodoacetate $\geq$ 98% | Sigma Aldrich (Merck) | SLBZ7569 |

**Table 8:** Induction Agents used in the Example 4 *in vivo* study.

| Treatment Name | Solute | Solvent | Concentration |
|---|---|---|---|
| Vehicle | N/A | PBS | N/A |
| MIA | MIA | PBS | 40 mg/ml (2 mg/50 $\mu$L) |

**Table 9:** Treatments used in the Example 4 *in vivo* study.

| Treatment Name | Solute | Solvent | Concentration | Notes |
|---|---|---|---|---|
| Vehicle | N/A | PBS | N/A | |
| B09P3.1 | PLA (1.9 kDa) | PBS | 13 mg/ml | • PLA from Alamanda Polymers See **Table 6** |
| B09P3.2 | PLA (1.9 kDa) | PBS | 13 mg/ml and 20 mg/ml | • Two different concentrations used • PLA from Alamanda Polymers • See **Table 6** |
| B09P3.3 | PLA (1.9 kDa) | PBS | 13 mg/ml and 20 mg/ml | • Two different concentrations used • PLA from Alamanda Polymers See **Table 6** |

**Animal Model**

All animals were Sprague-Dawley (SD) rats from Charles River UK were used in this study. The animals were maintained under standard laboratory conditions (temperature $22 \pm 2°C$, relative humidity 45 - 65%, 12:12-hour light/dark cycle with lights on from 08:00-20:00 h). The rats were acclimatised for a minimum of 7 days after arrival into the unit. No prophylactic or therapeutic treatment was administered during this acclimatisation period. The animals were group housed with 2-3 rats per cage in standard plastic bottom cages containing 3Rs basic bedding (3Rs Laboratory, UK), nesting material (Sizzle-Pet, LBS Biotechnology, UK), and a plastic tube. Food (14% protein rodent diet; Envigo, UK) and water were available ad libitum throughout the study.

**Intra-articular Injection**

Under brief isoflurane anaesthesia (2-3% in 0.8 L.min$^{-1}$ O$_2$), animals were injected once via the intra-articular (i.a.) route with one of the treatments as listed in Table 8 at a volume of 50 $\mu$L. Injections were administered using a 0.5 ml insulin syringe (30G; 8 mm). The needle was inserted perpendicularly through the infrapatellar ligament into the intra-articular space of the left tibiofemoral (knee) joint, with the contents of the syringe dispensed into the joint.

**Behavioural Tests**

Static Weight Bearing Test to Assess Weight Distribution Asymmetry

Static weight bearing (SWB) was assessed using an Incapacitance Tester (Linton Instrumentation, Norfolk, UK). This test measures primary hypersensitivity in OA-related chronic pain. The device has an angled plexiglass chamber designed to hold the rat with forepaws on the plexiglass, and hind paws on two separate force plates. The weight on each force plate is measured in grams. Calibration of both plates was done before each test session with a 50 g mass.

Rats were placed in the angled plexiglass chamber positioned so that each hind paw rested on a separate force plate. The force exerted by each hind limb (in grams) was averaged over a 3 s period.

For this example the distribution of weight borne on the hind limbs was reported as the percentage weight borne on the ipsilateral (injured/left) hind limb, calculated using the following equation:

$$\text{Percentage weight bearing} = \frac{\text{Weight on ipsilateral hind limb}}{\text{Weight on contralateral} + \text{ipsilateral hind limbs}} \times 100$$

Three successive readings were performed for each rat and the mean percentage weight borne on the ipsilateral hind limb was calculated.

von Frey Test to Assess Mechanical Sensitivity

Mechanical sensitivity was assessed using both the manual and electronic von Frey (VF) tests. These tests measure secondary hypersensitivity in OA-related chronic pain.

The electronic von Frey anaesthesiometer (eVF) from IITC Life Science Inc (Woodland Hills, CA, USA) was used here. The eVF was calibrated using the calibration weight (7.5 g) provided by the supplier. Once calibrated, a rigid tip was fitted to the probe. The eVF test was carried out 10 min after the manual VF test. The rigid tip was used to apply pressure to the plantar surface of the hind paw (same location as for the VF monofilaments) and the force was increased slowly until a positive response (brisk withdrawal, flinching, or licking of the paw) was elicited. The force at which this response occurs is recorded automatically by the apparatus and is designated as the PWT in grams. The test was repeated twice on each hind paw with an interval of at least 5 minutes. The average PWT for each hind paw per rat was then calculated.

**Study Design and Experimental Groups**

There were 7 experimental groups in this pilot study (Table 10).

**Table 10:** Experimental groups in in the Example 4 *in vivo* study.

| Group | Induction | Treatment | Route | Volume | n |
|---|---|---|---|---|---|
| 1 | PBS (Sham) | N/A | i.a | 50 μL | 6 |
| 2 | MIA 40 mg/ml | PBS | i.a | 50 μL | 6 |
| 3 | MIA 40 mg/ml | B09P3.2 (13 mg/ml) | i.a. | 50 μL | 6 |
| 4 | MIA 40 mg/ml | B09P3.3 (13 mg/ml) | i.a. | 50 μL | 6 |
| 5 | MIA 40 mg/ml | B09P3.1 (20 mg/ml) | i.a. | 50 μL | 6 |
| 6 | MIA 40 mg/ml | B09P3.2 (20 mg/ml) | i.a. | 50 μL | 6 |
| 7 | MIA 40 mg/ml | B09P3.3 (20 mg/ml) | i.a. | 50 μL | 6 |

Note: Choice of concentrations:

- 13 mg/ml: B05P03 (equivalent to B09P3.2) dynamic viscosity was found to be ~ 450 mPa.s at 13 mg/ml. This was approximately equivalent to HA at 10 mg/ml.
- 20 mg/ml: Found empirically to be the highest concentration achievable. Also, B05P03 (equivalent to B09P3.2) dynamic viscosity was found to be ~ 32000 mPa.s at 20 mg/ml. This was the same order of magnitude as Durolane® (20 mg/ml stabilised HA) at ~ 27000 mPa.s.

Randomisation: After acclimatisation, animals were tested for baseline behavioural responses on Days -5, -3, and -1 (SWB, eVF and AD tests) with respect to MIA injection. Three measurements were performed for each behavioural test to obtain stable baseline responses, and averaged. Animals were then randomly allocated to treatments groups resulting in 7 experimental groups. The experimenter (MF) was blind to treatment allocation.

Protocol:

The n = 42 animals (n = 6 per group) were divided into five blocks (n = 9, 6, 9, 9, 9 animals per block). Each block had a random allocation of animals from each experimental group. The time course of each animal was the same, with blocks staggered by one day.

On Day 0, rats received an i.a. injection of the assigned induction agent under brief isoflurane anaesthesia and allowed to recover in their home cages. Another i.a. injection of the assigned treatment agent was similarly administered on Day 14. Rats were assessed in the SWB, eVF, and AD tests (in that order) on Days 7, 11, 14 (+30 min post-treatment), 15, 28, 35, 42, and 63. Animals were euthanised on Day 64 by decapitation.

**Results**

**Behavioural Analysis: Static Weight Bearing**

The results of the SWB are shown in **Figure 7A** expressed as the percentage weight borne on the ipsilateral hind limb. MIA-injected rats showed a decrease in weight borne on the ipsilateral limb compared to the sham group, indicating MIA-induced weight bearing deficit.

Following administration of assigned treatments on Day 14, groups treated with the Hydrobloc prototypes did not show an improvement in this weight bearing deficit at an acute or early time point. However, from Day 35 onwards MIA-injected rats that were treated with Hydrobloc prototypes displayed clear and significant improvements in weight bearing deficit compared to vehicle-treated counterparts. Of note, the higher concentration (20 mg/ml) prototypes seemed to give better and sustained effects at these later time points than lower concentration (13 mg/ml) ones (not statistically significant).

**Behavioural Analysis: Electronic von Frey**

Behavioural results from the eVF test are summarised in **Figure 7C.** All experimental groups showed similar PWT at baseline on both sides. MIA-injected rats showed a decrease in PWT on ipsilateral hind paw compared to sham rats, indicating MIA-induced mechanical hypersensitivity **(Figure 7C).** Following administration of assigned treatments on Day 14, Hydrobloc-treated MIA rats did not show any improvement in mechanical hypersensitivity at an acute or early time point. However, a clear divergence in PWT between Hydrobloc- and vehicle-treated MIA rats was observed from Day 35 onward. There was significant improvement in mechanical hypersensitivity in the Hydrobloc-treated MIA rats on Day 42 compared to vehicle-treated counterparts. Trends for reduction in MIA-induced mechanical hypersensitivity were seen on Day 63 with some prototypes (notably B09P3.2 20 mg/ml and B09P3.3 20 mg/ml). The contralateral PWT did not differ between the groups at any time point

**Conclusions**

The results from this *in-vivo* study indicate that:

- Current Hydrobloc treatments **do acutely improve OA associated pain** in the MIA model as measured by weight distribution asymmetry (SWB), and mechanical sensitivity (eVF).
- Current Hydrobloc treatments **do however improve OA associated pain at longer term time points** in all tests, and most strikingly in the SWB test where a sustained effect seems to be present at these later time points.
- There is a suggestion that the **higher concentrations** (20 mg/ml) perform better, and possibly the prototypes with **increased levels of PLA,** but **neither parameter is significantly associated**

**with enhanced efficacy.**

In addition, the welfare score sheets demonstrate that the animals suffered no undue suffering or distress as assessed by the general and specific MIA-related distress parameters. Also, there was no occurrence of adverse events related to MIA injection or the treatment injections. These welfare results indicate the Hydrobloc treatments to be well tolerated and safe for injection as assessed in this study.

Finally, the treatments were judged easy to administer with good flow characteristics.

***Example* 5 - *In vivo* assessment**

Example 5 tested the efficacy and safety of different prototypes, and at different doses in the rat MIA model of OA.

Details on the prototypes, chemicals, induction agents, and prototypes referred to in this Example are provided in Tables 11, 12, 13, and 14 respectively.

**Table 6:** Composition of prototypes tested in the Example 5 *in vivo* study.

| Prototype Code: | HA | PLA |
|---|---|---|
| B05P01 | MW: 2200-2400 kDa<br>1g (0.434 $\mu$mol) | MW: 15 - 70 kDa<br>50 mg (1.176 $\mu$mol) |
| B05P02 | MW: 2200-2400 kDa | MW: 9.6 kDa |

(continued)

| Prototype Code: | HA | PLA |
|---|---|---|
| | 1g (0.434 $\mu$mol) | 11.14 mg (1.176 $\mu$mol) |
| B05P03 | MW: 2200-2400 kDa<br>1g (0.434 $\mu$mol) | MW: 1.9 kDa<br>2.2 mg (1.176 $\mu$mol) |

**Table 12:** Composition of chemicals used in the Example 5 *in vivo* study.

| Chemical Name | Abbreviated Name | Constituents | Company | Batch Number |
|---|---|---|---|---|
| Phosphate Buffered Saline | PBS | Sodium chloride 9mg; Potassium Dihydrogen Phosphate 0.03mg; Disodium Hydrogen Phosphate Dihydrate (Merck) 0.14mg; Water for Injection q.s. 1ml | Sigma Aldrich (Merck) unless stated | N/A |
| Hyaluronic Acid | HA | HA 2200-2400kDa | Contipro | 219-32749 |
| Monosodium iodoacetate | MIA | Sodium iodoacetate | | |

**Table 13:** Composition of induction agents used in the Example 5 *in vivo* study.

| Induction Agent Name | Solute | Solvent | Concentration | Notes |
|---|---|---|---|---|
| Saline | NaCl | Water for Injection | N/A | |
| MIA | MIA | Saline | 60 mg/ml | Total dose administered = 3 mg |

**Table 14:** Composition of treatments used in the Example 5 *in vivo* study.

| Treatment Name | Solute | Solvent | Concentration | Notes |
|---|---|---|---|---|
| Vehicle | N/A | PBS | N/A | |
| Unmodified HA | HA | PBS | 10 mg/ml | |
| B05P01 | HA + PLA (15 kDa - 70 kDa) | PBS | 10 mg/ml | PLA from Sigma-Aldrich |
| B05P02 | HA + PLA (9.6 kDa) | PBS | 10 mg/ml | PLA from Alamanda Polymers |
| B05P03 | HA + PLA (1.9 kDa) | PBS | 10 mg/ml | PLA from Alamanda Polymers |

**Animals**

Male Sprague Dawley rats (170-190 g on arrival, 5-6 weeks old) from Envigo RMS Israel were used in this study and maintained under standard laboratory conditions (temperature 20 ± 3°C, relative humidity 30 - 70%, 12:12-hour light/dark cycle, 15-30 air changes/h). The rats were acclimatised for at least 5 days after arrival into the unit. No prophylactic or therapeutic treatment was administered during this acclimatisation period. The animals were group housed with a maximum of 3 rats per cage in a polypropylene cage fitted with solid bottoms and with sterile wood shavings as bedding. Animals were provided ad libitum with a commercial, sterile rodent diet and had free access to drinking water supplied to each cage via polyethylene bottles with stainless steel sipper tubes.

**Intra-articular Injection**

Animals were injected twice via the intra-articular (i.a.) route: first one of the induction agents as listed in **Table 13** and subsequently 14 days later with one of the treatments as listed in **Table 14.** Animals were briefly anesthetised for i.a. injection using isoflurane and oxygen mixture maintained at low concentrations (2-3%) with exposure time between 2-3 min. All injections (induction agents and treatments) were administered into the left knee joint of the rat using a 30G needle at a volume of 50 μL.

**Behavioural Tests**

The Dynamic Weight Bearing (DWB) and von Frey (VF) tests were used to assess behaviour in this study.

Dynamic Weight Bearing Test to Assess Weight Distribution Asymmetry

The DWB test assesses spontaneous pain in the paws of the rats that is a measure of primary hypersensitivity in OA joint. The animal was placed in a transparent cage and allowed to freely move inside the cage. A matrix comprising around 2000 high precision force sensors was embedded in the floor of the enclosure. The force sensors measured the weight distribution on each of the four paws of the animal in grams for a single duration of 5 minutes. The animal was filmed from above using a high-definition camera. The video feed was analysed in real-time during the test using a tracking software allowing a precise analysis of the animal's posture. The weight distribution on each paw and the time spent on four paws was measured. The DWB was performed on study Days -1 (baseline), 10 (inclusion), 14 (30 min post-dosing, immediately after VF test), 15, 21, and 28.
Note: The test duration was 5 minutes, in which the behaviour of the animal was recorded. Analysis was performed using a video taken during the test in which the hind paws were identified. At least 2 minutes out of the total 5 minutes were analysed. Once the analysis was completed, the software provides the weight in grams the animal put on each of the hind paws as the main readout. A ratio between the injured (left/ipsilateral) and intact (right/contralateral) hind paws was then calculated (this is the method used by the CRO to report results from DWB test). The data was further analysed as the difference in weight borne between intact and injured hind paws. A decrease in the ratio or an increase in the difference in weight borne between the two hind paws indicates weight bearing deficit (or asymmetry) in the injured hind limb.

von Frey Test to Assess Mechanical Hypersensitivity

The VF test was used to assess mechanical allodynia in the rat paw. This test was used here as a measure of **secondary hypersensitivity** in OA. The animal was placed in an enclosure and positioned on a metal mesh surface, but allowed to move freely. The rat cabins were covered with red cellophane to diminish environmental disturbances. The test began after cessation of exploratory behaviour. The Touch Test™ Sensory Evaluator was used on the plantar surface of the left (ipsilateral) hind paw. The animal indicated sensation by pulling back its hind paw. The minimal force in grams needed to elevate the withdrawal reflex was considered as the value of reference. Decrease in force needed to induce a withdrawal reflex was indicative of mechanical allodynia. The VF test was performed on study Days - 1 (baseline), 10 (before grouping), 14 (30 min post-dosing), 15, 21, and 28.
Note: The VF test was started with the 4g filament. Stimulation was performed a few times, and if a clear response was seen, the force of the filament was designated as the mechanical withdrawal response. If there was no response, a higher filament was used. The highest filament tested was 26g. If there was no response with the 26g filament, the withdrawal threshold was set at 26g. Therefore, the VF filaments used during testing were 4g, 6g, 8g, 10g, 15g, and 26g.

**Design and Experimental Groups**

There were 6 experimental groups in this study, with n = 10 animals in each group **(Table 15):**

Table 15. Experimental groups used in the Example 5 *in vivo* study.

| Group | Induction | Treatment | Route | Volume (μL) | n |
|-------|-----------|-----------|-------|-------------|---|
| 1 | Saline | PBS | i.a. | 50 | 10 |
| 2 | MIA (3mg) | PBS | i.a. | 50 | 10 |
| 3 | MIA (3mg) | HA (10 mg/ml) | i.a. | 50 | 10 |
| 4 | MIA (3mg) | B05P01 (10 mg/ml) | i.a. | 50 | 10 |
| 5 | MIA (3mg) | B05P02 (10 mg/ml) | i.a. | 50 | 10 |
| 6 | MIA (3mg) | B05P03 (10 mg/ml) | i.a. | 50 | 10 |

Randomisation: During the acclimatisation period, animals were randomly assigned to experimental groups. Each dosing group was kept in separate cages to avoid cross-contamination that can occur through consumption of faecal matter. Additionally, following the DWB test on Day 10, the data was analysed and animals that showed differences between the hind paws compared to baseline were included and further allocated to the treatment groups. The aim was to allocate animals to have similar mean of ratio between the hind paws in all groups (based on DWB scores). Throughout the study, the person who tested the animals was blinded to the treatment groups.

Protocol: The protocol is outlined in **Table 16.**

**Table 16:** Protocol used in the Example 5 *in vivo* study.

| Day | Procedure |
|---|---|
| Day -2 | • Habituation to Von Frey test |
| Day -1 | • VF (Baseline)<br>• DWB (Baseline) |
| Day 0 | • Body weight measurements<br>• i.a. left knee injection of MIA (Groups 2-6) or saline (Group 1) |
| Day 7 | • Body weight measurements |
| Day 10 | • VF test<br>• DWB test<br>• Group selection |
| Day 14 | • Body weight measurements<br>• Dosing (i.a. left knee injection)<br>• Wait 30 minutes<br>• VF test<br>• DWB test |
| Day 15 | • VF test<br>• DWB test |
| Day 21 | • Body weight measurements<br>• VF test<br>• DWB test |
| Day 28 | • Body weight measurements<br>• VF test<br>• DWB test<br>• Termination |

**Results**

**Behavioural Analysis: von Frey Test**

VF results expressed as the paw withdrawal threshold (PWT).

**Figure 6A-B** shows the behavioural results from the VF test. There was no difference in the paw withdrawal thresholds (PWT) on the ipsilateral (left) hind paw in all the experimental groups at baseline. MIA-injected rats exhibited lower PWT at all post-MIA time points relative to baseline, but the difference did not reach statistical significance versus saline-injected rats. HA injection did not affect the ipsilateral PWT in the MIA rats. Interestingly, Hydrobloc B05P03 increased the PWT in MIA rats on Day 28, compared with vehicle-treated counterparts (MIA+B05P03 vs MIA+PBS: $p=0.017$). Similarly, Hydrobloc B05P01 produced a strong trend in increasing the PWT in MIA rats on Day 28 (MIA+B05P01 vs MIA+PBS: $p=0.072$). No such effect of the prototype B03P02 was observed in the MIA rats on Day 28.

**Behavioural Analysis: Dynamic Weight Bearing**

DWB results expressed as difference in the weight borne on the contralateral and ipsilateral (injured) hind limbs **(Contra - Ipsi).**

As shown in **Figure 6C** MIA injection increased the difference in weight borne between the intact and injured hind paws in the rats, indicating MIA-induced weight bearing asymmetry in the ipsilateral hind limb [MIA vs Saline (Day 10): p<0.001, t-test]. **Figure 6D** depicts the effects of treatment in the MIA rats. HA overall lowered weight bearing asymmetry in MIA rats compared to vehicle-treated counterparts [MIA+HA vs MIA+PBS (Day 15): p=0.03]. Hydrobloc prototypes B05P01 and B05P03, but not B05P02, produced a strong trend in reducing MIA-induced weight bearing asymmetry on Day 15 (MIA+B05P01 vs MIA+PBS: p=0.09; MIA+B05P03 vs MIA+PBS: p=0.08) but not at later time points.

**Conclusions**

The results from this *in vivo* study suggests that the prototypes B05P01, B05P02, and B05P03 - at the doses administered produced strong trends in improving weight bearing deficit in the OA joint in rats. In addition, the prototype B05P03 two weeks post-dosing demonstrated a clear antinociceptive effect evidenced by the increase in PWT in OA rats compared to vehicle-treated counterparts. B05P01 also showed strong trend in increasing PWT in OA rats whereas B05P02 did not have any effect on PWT two weeks post-administration. Taken together, these data indicates that the Hydrobloc prototype B05P03 exhibited potential in relieving both primary and secondary OA-related pain hypersensitivities in the rat MIA model of OA.

**Example 6 - Various Synthesis methods of formulations including Hydrobloc**

1. The synthesis of Hydrobloc was done by carbodiimide coupling reaction between hyaluronic acid (HA) and Poly-l-arginine (PA). Briefly 1% (w/v) of HA was dissolved overnight at room temperature (20°C) of 0.1 M MES buffer solution (pH 6.5). Next, 25 mmol of EDC and NHS was added drop-wise to the HA solution and stirred for 45 min at room temperature to activate the carboxyl groups of the HA. Followed by, PA (1.16 $\mu$mol) was added drop-wise to the activated HA solution. The total volume of reaction solution was made up to 900 ml with MES buffer (pH 6.5). The reaction was then allowed to proceed by stirring for 24 h at room temperature to obtain the final product of PA functionalised HA. After completion of the stirring, the solution was then dialysed (MWCO 100 kDa) against deionised water for 96 hrs and after that centrifuged the dialysed sample at 5000 RPM for 10 min at 4°C to precipitate any colloidal suspension. Following centrifugation, the solution was poured into freeze-drying bottles and frozen at -80°C for overnight. The frozen sample was freeze dried for 160 hrs to obtain white sponge-like material and kept stored at 4°C until further use. The material can be solubilized with sodium chloride buffer solution (pH 7) before use.

2. Polyethelene glycol was conjugated with PA by esterification reaction. Briefly, 2.2 mg of PA was dissolved at room temperature (20°C) of 0.1 M MES buffer solution (pH 6.5). Next, 1g of PAG was mixed with the solution and dissolved completely. 25 mmol of EDC and NHS was added drop-wise to the PA-PEG and the total volume of reaction solution was made up to 900 ml with MES buffer (pH 6.5). The reaction was then allowed to proceed by stirring for 24 h at room temperature to obtain the final product of PA functionalised PEG. After completion of the stirring, the solution was then dialysed (MWCO 14 kDa) against deionised water for 96 hrs and after that centrifuged the dialysed sample at 5000 RPM for 10 min at 4°C to precipitate any colloidal suspension. Following centrifugation, the solution was poured into freeze-drying bottles and frozen at -80°C for overnight. The frozen sample was freeze dried for 160 hrs to obtain white sponge-like material and kept stored at 4°C until further use. The material can be solubilized with sodium chloride buffer solution (pH 7) before use.

3. The synthesis of Hydrobloc was done by carbodiimide coupling reaction between Alginic acid (AA) and Poly-l-arginine (PA). Briefly 1% (w/v) of AA was dissolved overnight at room temperature (20°C) of 0.1 M MES buffer solution (pH 6.5). Next, 25 mmol of EDC and NHS was added drop-wise to the HA solution and stirred for 45 min at room temperature to activate the carboxyl groups of the AA. Followed by, PA (1.16 $\mu$mol) was added drop-wise to the activated AA solution. The total volume of reaction solution was made up to 900 ml with MES buffer (pH 6.5). The reaction was then allowed to proceed by stirring at 700 rpm for 24 h at room temperature to obtain the final product of PA functionalised AA. After completion of the stirring, the solution was then dialysed (MWCO 50 kDa) against deionised water for 96 hrs and after that centrifuged the dialysed sample at 5000 RPM for 10 min at 4°C to precipitate any colloidal suspension. Following centrifugation, the solution was poured into freeze-drying bottles and frozen at -80°C for overnight. The frozen sample was freeze dried for 160 hrs to obtain white sponge-like material and kept stored at 4°C until further use. The material can be solubilized with sodium chloride buffer solution (pH 7) before use.

4. Chitosan was conjugated with PA by amide coupling reaction. Briefly, 2.2 mg of PA was dissolved at room temperature (20°C) of 0.1 M MES buffer solution (pH 6.5). Next, 1g of chitosan was mixed with the solution and dissolved completely. 25 mmol of EDC and NHS was added drop-wise to the PA-Chitosan mixture and the total volume of reaction solution was made up to 900 ml with MES buffer (pH 6.5). The reaction was then allowed to proceed by stirring for 24 h at room temperature to obtain the final product of PA functionalised Chitosan. After completion of the stirring, the solution was then dialysed (MWCO 14 kDa) against deionised water for 96 hrs and after that centrifuged the dialysed sample at 5000 RPM for 10 min at 4°C to precipitate any colloidal suspension. Following centrifugation, the solution was poured into freeze-drying bottles and frozen at -80°C for overnight. The frozen sample was freeze dried for 160 hrs to obtain white sponge-like material and kept stored at 4°C until further use. The material can be solubilized with sodium chloride buffer solution (pH 7) before use.

5. Dextran was conjugated with PA by esterification reaction. Briefly, 2.2 mg of PA was dissolved at room temperature (20°C) of 0.1 M MES buffer solution (pH 6.5). Next, 1g of dextran was mixed with the solution and dissolved completely. 25 mmol of EDC and NHS was added drop-wise to the PA-Chitosan mixture and the total volume of reaction solution was made up to 900 ml with MES buffer (pH 6.5). The reaction was then allowed to proceed by stirring for 24 h at room temperature to obtain the final product of PA functionalised Dextran. After completion of the stirring, the solution was then dialysed (MWCO 14 kDa) against deionised water for 96 hrs and after that centrifuged the dialysed sample at 5000 RPM for 10 min at 4°C to precipitate any colloidal suspension. Following centrifugation, the solution was poured into freeze-drying bottles and frozen at -80°C for overnight. The frozen sample was freeze dried for 160 hrs to obtain white sponge-like material and kept stored at 4°C until further use. The material can be solubilized with sodium chloride buffer solution (pH 7) before use.

**List of Amine groups to block sodium channel**

- **Poly-Arginine**
- **Poly-Lysine**
- **Poly-Histidine**
- Poly-Ornithine
- Poly-Asparagine
- Poly-Glutamine
- Arginine hydrochloride
- Lysine hydrochloride
- Histidine
- Ornithine
- Asparagine
- Glutamine

**Claims**

1. A peptide drug for use in the localised modulation of nerve cell activity, cardiomyocyte or muscle cell electrical activity in a subject, wherein the peptide drug comprises a polymer in a carrier, and further comprising:

   i) a plurality of cationic peptides, or an amino acid monomer thereof, covalently bound to the polymer; and/or
   ii) a plurality of cationic peptides, or an amino acid monomer thereof, mixed with the polymer.

2. The peptide drug according to claim 1, wherein the polymer comprises or consists of a polymer selected from any of the group comprising polyacrylamide; pectin; alginate; carboxymethylcellulose; methylcellulose; PLGA; PEG; polysaccharide, such as starch, cellulose, chitin, alginate, hyaluronate; proteins such as collagen, gelatin, casein, albumin; polyvinyl alcohol (PVA); polyvinylpyrrolidone (PVP); polyethyleneglycol (PEG); polylactic acid; and polyhydroxy acid (PHA), or combinations thereof. The polymer may comprise or consist of a polymer selected from any of the group comprising poly-[alpha]-hydroxyacids including poly-lactide-co-glycolide (PLGA), poly-lactic acid, polyethyleneimine (PEI), polylactic or polyglycolic acids, poly- lactide poly-glycolide copolymers, and poly-lactide poly-glycolide polyethylene glycol copolymers, polyethylene glycol (PEG), polyesters, poly ([epsilon]-caprolactone), poly (3- hydroxy-butyrate) , poly (s-caproic acid), poly (p-dioxanone), poly (propylene fumarate), poly (orthoesters), polyol/diketene acetals addition polymers, polyanhydrides, poly (sebacic anhydride) (PSA), poly (carboxybiscarboxyphenoxyphosphazene) (PCPP), poly [bis(p-carboxyphenoxy) methane] (PCPM), poly (amino acids), poly (pseudo amino acids), polyphosphazenes, derivatives of poly [(dichloro) phosphazene], poly [(organo) phosphazenes], polyphosphates, polyethylene glycol polypropylene block co-polymers, natural or synthetic polymers such as silk, elastin, chitin, chitosan, fibrin, fibrinogen, polysaccharides (including pectins), glycosaminoglycans, alginates, collagen, peptides, polypeptides or proteins, copolymers prepared from the monomers of any of these polymers, random blends of these polymers, any suitable polymer and mixtures or combinations thereof.

3. The peptide drug according to claim 1, wherein the polymer comprises or consists of hyaluronic acid (HA) or sodium hyaluronate or polyethylene glycol (PEG).

4. The peptide drug according to any preceding claim, wherein the polymer has a molecular weight of at least 5 kDa.

5. The peptide drug according to any preceding claim, wherein the polymer is cross-linked.

6. The peptide drug according to any preceding claim, wherein the cationic peptide comprises or consists of CARPs (Cationic Arginine Rich Peptides).

7. The peptide drug according to any preceding claim, wherein the cationic peptide comprises or consists of one or more of arginine, and/ or lysine, and/ or histidine residues.

8. The peptide drug according to any preceding claim, wherein the cationic peptide, or monomer thereof, has a molecular weight in the range and of the order of $10^1$ Da to $10^4$ Da.

9. The peptide drug according to any preceding claim, wherein the peptide drug comprises or consists of:

   i) PEG polymer covalently linked to poly-arginine or poly-lysine; or combinations thereof;
   ii) hyaluronic acid polymer covalently linked to poly-arginine or poly-lysine or other cationic amino acid residues; or combinations thereof; or
   iii) hyaluronic acid polymer covalently linked to arginine monomer or lysine monomer or other cationic amino acid monomer; or combinations thereof.

10. The peptide drug according to any preceding claim, wherein the peptide drug is in the form of a gel.

11. The peptide drug according to any preceding claim, wherein the peptide drug is, or is arranged to be, administered to the subject by local injection or topical administration.

12. The peptide drug according to any preceding claim, wherein the peptide drug is administered by injection, with an injection volume of about 1 - 10ml and a dose of about 1 mg/ml - 30mg/ml.

13. A peptide drug comprising a polymer in a carrier, and further comprising:

   i) a plurality of cationic peptides, or an amino acid monomer thereof, covalently bound to the polymer; and/or
   ii) a plurality of cationic peptides, or an amino acid monomer thereof, mixed with the polymer.

14. A method of manufacturing the peptide drug according to claim 13, the method comprising the step of covalently bonding cationic peptides to the polymer and forming a gel, colloid, or solution in the carrier.

15. A method of treatment of a subject for pain or a motor neuron disorder selected from spasticity, migraine, dystonia or essential tremor, the method comprising the local administration of a peptide drug to a subject at the site of the pain or motor neuron disorder, wherein the peptide drug comprises a polymer in a carrier, wherein the polymer comprises a plurality of cationic peptides covalently bound to the polymer and/or cationic peptides mixed with the polymer.

16. Use of the peptide drug according to claim 13 for pain relief, motor neuron modulation or cardiomyocyte modulation in a subject.

17. Use of the peptide drug according to claim 13 for treatment of conditions related to abnormal activity of muscle cells.

18. A kit comprising the peptide drug according to claim 13, and a syringe.

**19.** Cationic peptides, such as Cationic Arginine-Rich Peptides (CARPs), for use in the localised modulation of nerve cell activity, cardiomyocyte or muscle cell electrical activity in a subject, optionally wherein the use comprises the administration of the cationic peptides to the subject by localised injection into a tissue or joint at the site of the pain and/or at the site of a motor neuron condition and/or the site of the muscle cells, and further optionally wherein the cationic peptides are administered at a dose of 1 - 10ml and about 1 microg/ml to 1500 microg/ml of cationic peptides.

**20.** The peptide drug according to claim 13 or cationic peptides, such as Cationic Arginine-Rich Peptides (CARPs), for use in the regeneration or cartilage tissue and/or the prevention of cartilage degeneration and/or protection of chondrocytes.

**21.** The peptide drug according to claim 13 or cationic peptides, such as Cationic Arginine-Rich Peptides (CARPs), for use in the treatment of osteoarthritis.

**22.** A method of treatment or prevention of osteoarthritis, the method comprising the local administration of the peptide drug according to claim 13 or cationic peptides, such as Cationic Arginine-Rich Peptides (CARPs) into a joint.

**23.** A method of enhancement of chondrocyte activity for cartilage regeneration or maintenance, or for regeneration or maintenance of a component thereof, such as collagen II,
wherein the method comprises administering the peptide drug according to claim 13 or cationic peptides, such as Cationic Arginine-Rich Peptides (CARPs) to chondrocytes.

Figure 1

Figure 2

EP 4 180 032 A1

**Figure 3**

A — Peptide-Drug

B — Peptide (PLA 1.9 kDa)

C

**Figure 4:**

A

% alueuhair activity

| Time-point | Cells | P01 | P02 | P03 | C01 | C02 | C03 | C04 | HA | Control |
|---|---|---|---|---|---|---|---|---|---|---|
| 24 hrs | DRG | 90 | 95 | 99 | 10 | 86 | 106 | 93 | 89 | 100 |
| | hChon | 106 | 100 | 98 | 6 | 103 | 107 | 97 | 96 | 100 |
| | OA | 99 | 101 | 100 | 5 | 98 | 101 | 94 | 94 | 100 |
| 48 hrs | DRG | 92 | 94 | 97 | 9 | 86 | 102 | 90 | 89 | 100 |
| | hChon | 91 | 90 | 89 | 4 | 90 | 95 | 78 | 82 | 100 |
| | OA | 93 | 96 | 95 | 5 | 91 | 104 | 91 | 87 | 100 |
| 72 hrs | DRG | 92 | 94 | 95 | 6 | 81 | 103 | 88 | 87 | 100 |
| | hChon | 100 | 98 | 102 | 6 | 105 | 115 | 96 | 93 | 100 |
| | OA | 92 | 93 | 97 | 4 | 90 | 96 | 88 | 91 | 100 |

B

| Time-point | Cells | Average Number of Dead Cells | | | | | | | | |
|---|---|---|---|---|---|---|---|---|---|---|
| | | P01 | P02 | P03 | C01 | C02 | C03 | C04 | HA | Control |
| 24 hrs | DRG | 145 | 158 | 121 | 374 | 309 | 213 | 222 | 207 | 157 |
| | hChon | 80 | 57 | 51 | 518 | 385 | 34 | 405 | 38 | 51 |
| | OA | 48 | 46 | 54 | 823 | 576 | 51 | 439 | 62 | 34 |
| 48 hrs | DRG | 183 | 261 | 255 | 497 | 430 | 96 | 318 | 72 | 274 |
| | hChon | 76 | 51 | 49 | 754 | 357 | 66 | 374 | 57 | 18 |
| | OA | 153 | 146 | 98 | 827 | 489 | 185 | 683 | 150 | 30 |
| 72 hrs | DRG | 219 | 287 | 204 | 475 | 509 | 449 | 313 | 148 | 318 |
| | hChon | 148 | 74 | 61 | 763 | 287 | 73 | 464 | 95 | 30 |
| | OA | 378 | 192 | 151 | 857 | 300 | 158 | 601 | 238 | 79 |

# Figure 5

A

| Viscosity Testing | | | | | | | | |
|---|---|---|---|---|---|---|---|---|
| | 10 mg/ml | | | | 20 mg/ml | | | |
| Sample | Shear Rate (1/s) | Speed (RPM) | Torque (%) | Dynamic Visc. (mPa. S) | Shear rate (1/s) | Speed RPM | Torque (%) | Dyn. Visc. mPa. S |
| HA 2.2 – 2.4 MDa | 322.5 | 250 | 91.3 | 443.4 | / | / | / | / |
| HA 0.75 MDa | 322.5 | 250 | 32.8 | 159.2 | 2.582 | 2 | 53.5 | 8612 |
| HB P01 | 322.5 | 250 | 28.7 | 139.3 | | | | |
| HB P02 | 322.5 | 250 | 43.7 | 212.2 | 2.582 | 2 | 52.9 | 32080 |
| HB P03 | 322.5 | 250 | 43.6 | 211.8 | 2.582 | 2 | 52.5 | 31840 |
| HB P03.3 | 322.5 | 250 | 42.5 | 206.3 | 2.582 | 2 | | |
| HB P03.1 | 322.5 | 250 | 48.2 | 233.8 | 2.582 | 2 | | |
| HB P03.12 | 322.5 | 250 | 33.7 | 163.5 | 2.582 | 2 | 35.9 | 21790 |
| HB P03.13 | 322.5 | 250 | 14.7 | 71.37 | 2.582 | 2 | 5.73 | 3477.3 |
| Durolane | / | / | / | / | 2.582 | 2 | 45.2 | 27410 |

B

C

EP 4 180 032 A1

# Figure 6

**A**

**p<0.01 (MIA vs Saline)

**B**

+p<0.05 (MIA+ HBP03 vs MIA+PBS)

Data=Mean ± SEM
n=9-10/group

**C**

**D**

EP 4 180 032 A1

# Figure 7

## Figure 8:

# Von Frey test

**A**

Wilcoxon test: *p<0.01 (30 min vs baseline)

n=13-14/group

## 30 min

**B**

One way ANOVA, *posthoc* Fisher's LSD test

n=13-14/group

*p<0.05 (hydrobloc vs saline)

Data=mean ± SEM

**Figure 9**

Enzymatic degradation comparison between Commercial HA & HYDROBLOC

EP 4 180 032 A1

Figure 10:

# Figure 11

**A**

Patch Clamp Sodium current – HB B10-PEG 1 mg/ml
n = 2; motor neuron

**B**

EP 4 180 032 A1

**Figure 12:**

**A**

*in-vitro* evidence

Chondrocytes
(control)

Chondrocytes
with HB P01
(Day 3)

HB P01 promotes chondrocyte proliferation
(nuclei stained blue) and increased
production of Collagen II (stained green):
x17 times more by Day 3

**B**

*in-vivo* evidence

MIA + Saline
(Day 28 Post-
MIA)

MIA + HB P03.3
(Day 28 Post-MIA)
**\*Increased
cellularity**

**EP 4 180 032 A1**

## EUROPEAN SEARCH REPORT

Application Number

EP 21 20 8639

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | VEDADGHAVAMI ARMIN ET AL: "Cartilage penetrating cationic peptide carriers for applications in drug delivery to avascular negatively charged tissues", ACTA BIOMATERIALIA, ELSEVIER, AMSTERDAM, NL, vol. 93, 15 July 2019 (2019-07-15), pages 258-269, XP085730271, ISSN: 1742-7061, DOI: 10.1016/J.ACTBIO.2018.12.004 [retrieved on 2018-12-06] * the whole document * * abstract * | 1-14 | INV. A61K31/04 A61K31/16 A61K31/198 A61K31/728 A61K47/61 A61K47/64 A61P21/00 A61P25/00 A61P29/00 |
| Y | ZHANG XIAOWEI ET AL: "Exploiting Joint-Resident Stem Cells by Exogenous SOX9 for Cartilage Regeneration for Therapy of Osteoarthritis", FRONTIERS IN MEDICINE, vol. 8, 12 February 2021 (2021-02-12), XP055944792, DOI: 10.3389/fmed.2021.622609 * the whole document * * abstract; compound scSOX9 * | 1-14 | TECHNICAL FIELDS SEARCHED (IPC) A61K A61P |

-/--

The present search report has been drawn up for all claims

| Place of search | Date of completion of the search | Examiner |
|---|---|---|
| Munich | 25 July 2022 | Orlando, Michele |

EPO FORM 1503 03.82 (P04C01)

## EUROPEAN SEARCH REPORT

Application Number

EP 21 20 8639

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LEBEDEV DMITRY S. ET AL: "Oligoarginine Peptides, a New Family of Nicotinic Acetylcholine Receptor Inhibitors", MOLECULAR PHARMACOLOGY, vol. 96, no. 5, 1 November 2019 (2019-11-01), pages 664-673, XP055944231, US ISSN: 0026-895X, DOI: 10.1124/mol.119.117713 Retrieved from the Internet: URL:https://molpharm.aspetjournals.org/content/molpharm/96/5/664.full.pdf?with-ds=yes> * the whole document * * figures; tables; compounds W2R4, R6, R8, R16 * | 1-14 | |
| Y | HIBBITTS ALAN ET AL: "Poly(ethylene glycol)-Based Peptidomimetic "PEGtide" of Oligo-Arginine Allows for Efficient siRNA Transfection and Gene Inhibition", ACS OMEGA, vol. 4, no. 6, 10 June 2019 (2019-06-10), pages 10078-10088, XP055944058, US ISSN: 2470-1343, DOI: 10.1021/acsomega.9b00265 Retrieved from the Internet: URL:http://pubs.acs.org/doi/pdf/10.1021/acsomega.9b00265> * the whole document * * compounds * | 1-14 | TECHNICAL FIELDS SEARCHED (IPC) |

-/--

The present search report has been drawn up for all claims

| Place of search | Date of completion of the search | Examiner |
|---|---|---|
| Munich | 25 July 2022 | Orlando, Michele |

Europäisches
Patentamt
European
Patent Office
Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TSIOURVAS DIMITRIS ET AL: "Insulin complexes with PEGylated basic oligopeptides", JOURNAL OF COLLOID AND INTERFACE SCIENCE, vol. 384, no. 1, 1 October 2012 (2012-10-01), pages 61-72, XP055944062, US ISSN: 0021-9797, DOI: 10.1016/j.jcis.2012.06.068 * the whole document * * compounds * | 1-14 | |
| Y | WO 2004/004744 A1 (PERICOR SCIENCE INC [US]; SVIRKIN YURI [US] ET AL.) 15 January 2004 (2004-01-15) * the whole document * * claims; examples * | 1-14 | |
| Y | MELONI BRUNO P. ET AL: "Cationic Arginine-Rich Peptides (CARPs): A Novel Class of Neuroprotective Agents With a Multimodal Mechanism of Action", FRONTIERS IN NEUROLOGY, vol. 11, 25 February 2020 (2020-02-25), page 108, XP055873065, ISSN: 1664-2295, DOI: 10.3389/fneur.2020.00108 * the whole document * * Supplementary Material: Table 2. * | 1-14 | TECHNICAL FIELDS SEARCHED (IPC) |

-/--

The present search report has been drawn up for all claims

| Place of search | Date of completion of the search | Examiner |
|---|---|---|
| Munich | 25 July 2022 | Orlando, Michele |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

## EUROPEAN SEARCH REPORT

Application Number

EP 21 20 8639

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BATULU HU ET AL: "Effect of poly-arginine R18 on neurocyte cell growth via autophagy in traumatic brain injury", EXPERIMENTAL AND THERAPEUTIC MEDICINE, vol. 17, 20 March 2019 (2019-03-20), pages 4109-4115, XP055929296, GR ISSN: 1792-0981, DOI: 10.3892/etm.2019.7423 * the whole document * | 1-14 | |
| A | GUPTA SACHCHIDANAND SOAHAM ET AL: "Amino acid derived biopolymers: Recent advances and biomedical applications", INTERNATIONAL JOURNAL OF BIOLOGICAL MACROMOLECULES, ELSEVIER BV, NL, vol. 188, 10 August 2021 (2021-08-10), pages 542-567, XP086776253, ISSN: 0141-8130, DOI: 10.1016/J.IJBIOMAC.2021.08.036 [retrieved on 2021-08-10] * the whole document * | 1-14 | |
| A | JP 3 616344 B2 (NISHIMURA SHINICHIRO; HOKKAIDO ELECTRIC POWER ET AL.) 2 February 2005 (2005-02-02) * the whole document * * paragraph [0013] * | 1-14 | |

TECHNICAL FIELDS SEARCHED (IPC)

The present search report has been drawn up for all claims

| Place of search | Date of completion of the search | Examiner |
|---|---|---|
| Munich | 25 July 2022 | Orlando, Michele |

Europäisches
Patentamt

European
Patent Office

Office européen
des brevets

**Application Number**

**EP 21 20 8639**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-14**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

The Search Division considers that the present European patent application does not comply with the requirements of unity of invention and relates to several inventions or groups of inventions, namely:

1. claims: 1-14

   A peptide drug according to claim 13 and its use according to claim 1 for the localised modulation of nerve cell activity, cardiomyocyte or muscle cell electrical activity. A method of manufacturing said peptide according to claim 14.

   ---

2. claim: 15

   A method of treating pain or a motor neuron disorder in a patient using the peptide drug.

   ---

3. claim: 19

   Cationic peptides, such as Cationic Arginine-Rich Peptides (CARPs), for use in the localised modulation of nerve cell activity, cardiomyocyte or muscle cell electrical activity in a subject.

   ---

4. claim: 20 (partially)

   The peptide drug for use in regeneration of cartilage tissue and/or the prevention of cartilage degeneration and/or protection of chondrocytes.

   ---

5. claim: 20 (partially)

   The cationic peptides, such as CARPs, for use in regeneration of cartilage tissue and/or the prevention of cartilage degeneration and/or protection of chondrocytes.

   ---

6. claim: 21 (partially)

   The peptide drug for use in the treatment of osteoarthritis.

   ---

7. claim: 21 (partially)

   The cationic peptides, such as CARPs, for use in the treatment of osteoarthritis.

   ---

## ANNEX TO THE EUROPEAN SEARCH REPORT
## ON EUROPEAN PATENT APPLICATION NO.

EP 21 20 8639

This annex lists the patent family members relating to the patent documents cited in the above-mentioned European search report.
The members are as contained in the European Patent Office EDP file on
The European Patent Office is in no way liable for these particulars which are merely given for the purpose of information.

25-07-2022

| Patent document cited in search report | Publication date | Patent family member(s) | Publication date |
|---|---|---|---|
| WO 2004004744 A1 | 15-01-2004 | AU 2003256381 A1 | 23-01-2004 |
| | | BR PI0312331 A2 | 28-06-2016 |
| | | CA 2491054 A1 | 15-01-2004 |
| | | CN 1671400 A | 21-09-2005 |
| | | CN 102178692 A | 14-09-2011 |
| | | CN 103638040 A | 19-03-2014 |
| | | EP 1539193 A1 | 15-06-2005 |
| | | JP 4818608 B2 | 16-11-2011 |
| | | JP 2006502988 A | 26-01-2006 |
| | | JP 2010270126 A | 02-12-2010 |
| | | MX PA05000186 A | 17-08-2005 |
| | | NZ 537735 A | 31-08-2006 |
| | | US 2006094643 A1 | 04-05-2006 |
| | | WO 2004004744 A1 | 15-01-2004 |
| JP 3616344 B2 | 02-02-2005 | JP 3616344 B2 | 02-02-2005 |
| | | JP 2002291461 A | 08-10-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LARRAÑETA et al.** *Carbohydrate Polymers,* 01 February 2018, vol. 181, 1194-1205
- **REISSIG JL ; STROMINGER JL ; LELOIR LF.** A modified colorimetric method for the estimation of N-acetylamino sugars. *J Biol Chem,* 1955, vol. 217, 959-96

- **PAVIA, D. L. ; LAMPMAN, G. M. ; KRIZ, G. S.** Introdução àespectroscopia. Cengage Learning, 2010, 700